# EUROPÄISCHE PATENTSCHRIFT

(11) **EP 3 013 937 B1**
(45) Veröffentlichungstag und Bekanntmachung des Hinweises auf die Patenterteilung: **15.05.2019**
(21) Anmeldenummer: 14744287.5
(22) Anmeldetag: 27.06.2014
(51) Int. Cl.: C12M 1/107, C12M 1/12, C12M 1/00

(54) **VERFAHREN ZUR BIOMETHANISIERUNG VON H2 UND CO2**
METHODS FOR THE BIOMETHANATION OF H2 AND CO2
PROCÉDÉS DE BIOMÉTHANISATION DE H2 ET DE CO2

(30) Priorität: 28.06.2013 DE 102013010826
(43) Veröffentlichungstag der Anmeldung: 04.05.2016
(73) Patentinhaber: Brunner, Matthias, 66121 Saarbruecken (DE)
(72) Erfinder: Brunner, Matthias, 66121 Saarbruecken (DE)
(74) Vertreter: Schiweck Weinzierl Koch Patentanwälte Partnerschaft mbB
(86) Internationale Anmeldenummer: PCT/EP2014/063719
(87) Internationale Veröffentlichungsnummer: WO 2014/207211

(56) Entgegenhaltungen:
- EP-A2- 1 574 581
- JP-A- H06 169 783
- KR-A- 20090 008 987
- US-A1- 2009 130 734
- US-A1- 2013 005 010
- GUIOT ET AL.: "Potential of Wastewater-Treating Anaerobic Granules for Biomethanation of Synthesis Gas", ENVIRONMENTAL SCIENCE TECHNOLOGY, Bd. 45, 3. Februar 2011 (2011-02-03), Seiten 2006-2011, XP055078669,

## Beschreibung

Eine Speicherung von Energie ist nach dem Konzept "power to gas" durch Erzeugung speicherbaren Methans möglich. Zunächst wird mit nicht genutzten Spitzen regenerativ erzeugter Energie per Elektrolyse Wasserstoff erzeugt. Aus Wasserstoff (H₂) kann zusammen mit Kohlendioxid (CO₂) speicherbares Methan (CH₄) hergestellt werden. Die Methanbildung lässt sich nach folgender Gleichung bilanzieren: 4 H₂ + CO₂ ⇔ CH₄ + 2 H₂O (sog. Sabatier-Reaktion). Diese Reaktion kann auf rein chemisch und auf biologischem Weg durchgeführt werden. Die chemische Reaktion wird bei Temperaturen von über 180°C und hohen Drücken erzwungen und wird durch relativ anspruchsvolle Katalysatoren ermöglicht.

Die biologische Umwandlung erfolgt durch spezielle Mikroorganismen. In natürlichen, anaeroben, wässrigen Ökosystemen, wie Sümpfe, Gewässersedimenten oder überfluteten Böden wird von Konsortien mehreren Organismengruppen über eine Abbaukette Methan aus organischem Material, wie Pflanzen- und Tierresten gebildet. Es gibt mesophile und thermophile Konsortien. Ihre Temperaturoptima liegen bei 30-40°C respektive 50-60°C. Dabei bilden die Methanbakterien das letzte Glied dieser Abbaukette, die organisches Substrat über viele Zwischenstufen, wie organische Säuren, Alkohole und Wasserstoff, zu Methan und Kohlendioxid abbauen. Methanbakterien zählen zu den Archaebakterien (Archaea). Sie leben nur unter sauerstofffreien Bedingungen, haben sehr spezifische Nährstoffbedürfnisse, wachsen sehr langsam und haben ein sehr eingeschränktes Substratspektrum. Einige Methanbakterien nutzen Essigsäure oder Ameisensäure zur Methanbildung. Andere Methanbakterien bilden Methan nach obiger Gleichung aus Wasserstoff und Kohlendioxid.

Bekannten technischen Anlagen zur biogenen Methanbildung sind zur Umsetzung gasförmiger Substrate nicht geeignet. Solche Anlagen, wie Biogasanlagen, Faultürme oder Anlagen zur anaeroben Abwasserbehandlung wurden ausschließlich daraufhin optimiert, hydrolysierbare oder gelöste organische Substrate über die besagte Abbauketten zu biomethanisieren. Auch Wasserstoff wird als Zwischenprodukt gebildet. Es erfordert eine sehr niedrigen Wasserstoffpartialdruck von kleiner 10⁻⁴ bar, um dabei entstehende organische Zwischenprodukte weiter abbauen zu können. Wasserstoff verbrauchenden Methanbakterien halten den nötigen geringen Wasserstoffpartialdruck aufrecht. Würde diesem Prozess Wasserstoff zugesetzt werden, könnten die gesamte Abbaukette geschädigt und zum Erliegen gebracht werden.

Soll mit Hilfe oben beschriebenen wasserstoffverwertenden Bakterien Methan hergestellt werden, sollte anders als in der obigen Abbaukette, ein möglichst hoher Wasserstoffpartialdruck herrschen, um die Bakterien mit ihren gasförmigen Substrat zu versorgen. Die den Methanbakterien in der Abbaukette vorgelagerten Organismengruppen spielen dann keine Rolle mehr. Es sollte also mehr Gas angeboten werden als die Bakterien in der Lage sind umzuwandeln, um eine maximale Umwandlungsgeschwindigkeit zu erreichen.

Zu Forschungszwecken werden im Labormaßstab (Reagenzgläser, Flaschen etc.) Reinkulturen solcher Organismen direkt über die Gasphase mit ihren Substraten versorgt. Die Organismen ziehen quasi die von ihnen verstoffwechselten Gase aus der Gasphase über einen Gradienten an sich und geben das gebildete Methan in die Gasphase ab. Solche Reinkulturen werden großtechnisch nicht eingesetzt, da sie als strikt anaerobe Lebewesen bereits von Spuren von Sauerstoff abgetötet werden. Bisher bestand hierzu auch kein Anlass. Großtechnisch müssen Organismen auf andere Weise begast werden.

Vor diesem Hintergrund und im Zuge neuer Energiespeicherkonzepte (power to gas) wurden Verfahren vorgeschlagen, Methanbakterien zur Umwandlung von Wasserstoff und Kohlendioxid einzusetzen, die auf eine Erhöhung der Gasaustauschoberfläche abzielen, Festbettreaktoren mit großer Gasphase. Geeignete Organismen wachsen dabei als Biofilme auf Trägermaterialien, werden mit Nährmedium überspült und aus der Gasphase versorgt. Nachteil dieser Verfahren ist, dass diese Oberflächen zunächst von den Organismen bewachsen werden müssen. Methanbakterien wachsen jedoch sehr langsam und haben selbst unter optimalen Bedingungen Generationszeiten von mehreren Tagen. Fraglich ist zudem, wie Methanbakterien überhaupt einen Biofilm bilden sollten. Die Anfahrphase solche Reaktoren ist in der Praxis sehr lang, unsicher und kaum zu steuern. Werden die Biofilme später zu dick, können innenliegende Organismen nur suboptimal versorgt werden und es ergeben sich "Totzonen". Die Biomassekonzentration pro Reaktorvolumen lässt sich so nur schwer steuern. Nach einer Schädigung der Organismen durch einen Störfall könnten solche Reaktoren nur langsam wieder in Betrieb genommen werden. Ein Einsatz dieser Verfahren in der Praxis ist nicht bekannt.

Aus der US-Patentanmeldung US 2009/(130734 A1 ist Verfahren zur Umwandlung von Kohlendioxid zu Methan bekannt, bei dem eine Kultur von hydrogenotrophen methanogenen Archaeen in einem Bioreaktor hergestellt wird dem Bioreaktor ein Ausgangsgas aus einem industriellen Prozess zugeführt wird; wobei das Ausgangsgas CO₂ und zwischen 0,02% und 6,7% Sauerstoff (Mol/Volumen Ausgangsgas) umfasst und wobei die hydrogenotrophen methanogenen Archaeen das Ausgangsgas umwandeln, um kontinuierlich Methan zu erzeugen.

In der koreanischen Druckschrift KR 2009 0008987 A ist ein Verfahren zur Herstellung von biologischem Methan aus Wasserstoff und Kohlendioxid beschrieben, um Kohlendioxid bei Raumtemperatur und normalem Druck durch Verwendung von organischem Abfall massiv zu reduzieren.

Ein weiteres Verfahren zur Herstellung von Methangas aus einem CO2-haltigen Ausgangsmaterial ist aus der europäischen Patentanmeldung EP 1 574 581 A2 bekannt. Darin wird ein Verfahren und eine Vorrichtung zur Herstellung von Methangas beschrieben, wobei ein CO2 enthaltendes Ausgangsmaterial auf eine umweltfreundliche Art und Weise in einen energiereichen Gasstrom umgewandelt wird.

In der US-Patentanmeldung US 2013/005010 A1 wird ein Bioreaktor beschrieben, der einen Hauptreaktor und einen Wachstumsreaktor aufweist. Ferner wird hier ein Verfahren beschrieben, bei dem acetogene Bakterien mit Synthesegas in einem Wachstumsfermenterabschnitt eines Reaktorbehälters in Kontakt gebracht werden, der mit einem Hauptfermenterabschnitt eines Reaktorbehälters kontinuierlich ausgebildet ist.

Aus dem Fachartikel "Potential of wastewater-treating anaerobic granules for biomethanation of synthesis gas" von Guiot et al., publiziert in Environmetnal Science Technology, Bd. 45, 3. Februar 2011, Seiten 2006-2011, ist ein Verfahren zur Umwandlung von Synthesegas in Biogas (hauptsächlich Methan) durch anaerobe Fermentation beschrieben. Dazu wird Granulatschlamm als Reaktorinokulum in einen 30-Liter-Gasliftreaktor eingebracht und mit CO versorgt, um die Produktion von Methan und anderen Metaboliten bei verschiedenen Gasverdünnungen sowie Einspeisungs- und Rezirkulationsgeschwindigkeiten zu untersuchen. Bei einem CO-Partialdruck von 0,6 atm, kombiniert mit einem Gasrückführungsverhältnis von 20: 1, wurde eine maximale CO-Umwandlungseffizienz von 75% festgestellt, die hinsichtlich des Gas-Flüssigkeits-Massentransfers beschränkt war.

Die japanische Patentanmeldung JPH 06169783 A offenbart die Erzeugung von Energie und einer nützlichen Substanz mit Mehrzweck- und Komplexfunktion durch stetiges Zuführen von Wasserstoff und Kohlendioxidgas, das von Wasserstoff assimilierenden methanbildenden Bakterien aus endogenen und exogenen Quellen benötigt wird.

Zusammenfassend lässt sich feststellen, dass die Erzeugung und Einspeisung von SNG (Substitute Natural Gas) ins Erdgasnetz eine vielversprechende Möglichkeit darstellt, Energie aus erneuerbaren Quellen unter Verwendung der bestehenden Erdgas-Infrastruktur zu speichern und mit hohen Wirkungsgraden zu verwerten. Bei der Methangasherstellung besteht aber noch Optimierungsbedarf.

Aufgabe der vorliegenden Erfindung ist es daher effektive und kostengünstige Mittel und Verfahren bereitzustellen, mit denen unter Verwendung von methanogenen Mikroorganismen Methan hergestellt werden kann.

Diese Aufgabe wird von der vorliegenden Erfindung gelöst, insbesondere wird die Aufgabe durch die nachstehenden Aspekte der vorliegenden Erfindung, wie Vorrichtungen, Verfahren oder Verwendungen und Ausführungsformen davon sowie durch die Gegenstände der Ansprüche gelöst. Die Abbildungen illustrieren die vorliegende Erfindung.

In einem ersten Aspekt betrifft die vorliegende Erfindung eine Vorrichtung zum Herstellen von Methan mittels methanogener Mikroorganismen durch Umsetzung von H₂ und CO₂, aufweisend:
- mindestens einen Reaktor;
- ein wässriges Medium, welches in dem mindestens einen Reaktor bereitgestellt ist, wobei sich die methanogenen Mikroorganismen in dem wässrigen Medium befinden;
- eine Zuführungseinrichtung, welche zum Einleiten von H₂ und CO₂ in den mindestens einen Reaktor eingerichtet ist, wobei das H₂ und CO₂ darin ein gasförmiges Gemisch bilden;
- eine Reaktionssteigerungsvorrichtung, welche eingerichtet ist zum Vergrößern der Kontaktoberfläche zwischen dem wässrigen Medium mit den methanogenen Mikroorganismen und dem gasförmigen Gemisch; und
- eine Rückführungseinrichtung, welche eingerichtet ist zum Rückführen mindestens eines Teils des sich im Reaktor ansammelnden Gases, wobei die Rückführungsrate des sich im Reaktor ansammelnden Gases größer ist als die Zuführungsrate des Substratgases in den Reaktor.

In einem zweiten Aspekt betrifft die vorliegende Erfindung die Verwendung einer erfindungsgemäßen Vorrichtung zur Herstellung von Methan mittels methanogener Mikroorganismen durch Umsetzung von H₂ und CO₂.

In einem dritten Aspekt betrifft die Erfindung Verfahren zur Herstellung von Methan in einer erfindungsgemäßen Reaktorvorrichtung mittels methanogener Mikroorganismen, die sich in der Reaktorvorrichtung in einem wässrigen Medium befinden, durch Umsetzung von H₂ und CO₂ als Reaktionsgasgemisch, wobei bei der Umsetzung die Kontaktoberfläche zwischen dem wässrigen Medium mit den methanogenen Mikroorganismen und dem gasförmigen Gemisch mittels einer Reaktionssteigerungsvorrichtung vergrößert wird und wobei mittels einer Rückführungseinrichtung mindestens ein Teils des sich im Reaktor ansammelnden Gases in die Reaktorvorrichtung zurückgeführt wird, wobei die Rückführungsrate des sich im Reaktor ansammelnden Gases größer ist als die Zuführungsrate des Reaktionsgemisches in den Reaktor.

Die vorliegende Erfindung betrifft also Vorrichtungen, Verfahren oder Verwendungen und Ausführungsformen davon, die nachstehend detaillierter beschrieben sind. Bevorzugte Ausführungsformen, die z.B. für Vorrichtungen beschrieben sind, treffen auch, *mutatis mutandis*, auf Verfahren bzw. Verwendungen zu, und umgekehrt, d.h. Ausführungsformen, die z.B. für Verfahren oder Verwendungen beschrieben sind, treffen auch, *mutatis mutandis*, auf Vorrichtungen zu.

Aus Wasserstoff (H₂) und Kohlendioxid (CO₂) kann chemisch und mikrobiell Methan (CH₄) erzeugt werden. Die chemische Reaktion erfolgt katalysiert bei über 180°C und hohem Druck. Entsprechend hoch sind dabei die Ansprüche an Material und Gerät. Mikrobiell werden H₂ und CO₂ in aquatischen Ökosystemen von Methanbakterien unter physiologischen Bedingungen unter Produktion von CH₄ verstoffwechselt. Diese Organismen lassen sich auch technisch nutze. Bekannte Anlagen zur Biomethanisierung (Biogasanlagen etc.) sind nur zum Abbau fester oder gelöster organischer Kohlenstoffverbindungen geeignet. Zugesetztes H₂ störte diesen Abbau. Technische Anlagen zur mikrobiellen Umsetzung von H₂ und CO₂ sind nicht bekannt. Die Versorgung mit H₂ limitiert den Stoffwechsel der Bakterien. Im Labormaßstab sind Submersreaktoren bekannt, sowie anaerobe Filter mit von Bakterien bewachsene Oberflächen. Methanbakterien besiedeln solche Oberflächen jedoch sehr langsam.

Vom Erfinder wurde nun festgestellt, dass eine effektive und kostengünstige Art der Herstellung von Methangas in Reaktoren, vorzugsweise Feststoffreaktoren, entgegen der Ansicht im Stand der Technik, möglich ist, sofern die methanogenen Mikroorganismen auf ideale Weise mit den Substraten Wasserstoff (H₂) und Kohlendioxid (CO₂) in Kontakt gebracht werden. Dazu regt der Erfinder an, die Kontaktoberfläche zwischen dem wässrigen Medium des Reaktors, in dem sich die methanogenen Mikroorganismen befinden, und dem gasförmigen Substratgasgemisch (gasförmiges Gemisch) auf geeignete Weise zu vergrößern, im Idealfall zu maximieren. Beispielsweise und bevorzugt wird die Kontaktoberfläche zwischen dem wässrigen Medium im Reaktor der erfindungsgemäßen Vorrichtung und dem gasförmigen Gemisch, das Kohlendioxid und Wasserstoff enthält, mittels einer Reaktionssteigerungsvorrichtung vergrößert.

Zur Vergrößerung der Kontaktoberfläche gab es im Stand der Technik die Anregung, methanogene Mikroorganismen in Form eines Biofilms auf und in einem Festbett, z.B. eingebracht als Füllkörper in einen Reaktor, wachsen zu lassen und dann mit H₂ und CO₂ zu umspülen, während ein Flüssigkeitsfilm über die im Biofilm befindlichen Mikroorgansimen läuft oder rieselt (DE 10 2011 051 836). Mit dieser Ausführung wollte man die hydrostatische Flüssigkeitssäule, die sich in einem Reaktor aufbaut umgehen, da man annahm, dass sie das Einbringen der Substratgase H₂ und CO₂ hindert bzw. dazu führt, dass sich insbesondere CO₂ im wässrigen Medium des Reaktors löst und somit nicht als Substratgas für die methanogenen Mikroorganismen zur Verfügung steht. Jedoch verkannte man, dass überhaupt ein Biofilm von methanogenen Mikroorganismen entstehen könnte, geschweige denn, wie lange es dauern würde, dass ein solcher Biofilm entsteht, so dass eine entsprechende Vorrichtung zum Herstellen von Methan mittels methanogener Mikroorganismen kostengünstig und effektiv betrieben werden könnte. Typischerweise haben methanogene Mikroorganismen selbst unter idealen Bedingungen eine Generationszeit von 3-5 Tagen. Zudem darf es weder in der Wachstumsphase noch später bei einer möglichen Stillzeit des Reaktors zu keinem Sauerstoffeintrag kommen, da die methanogenen Mikroorganismen strikt anaerob sind und sofort absterben würden. Eine Stillzeit der Vorrichtung wäre nicht ungewöhnlich, da es beim Projekt power to gas z.B. durchaus vorkommt, dass nur in Spitzenzeiten Strom dazu verwendet wird, Wasser elektrolytisch in Wasserstoff und Sauerstoff (O₂) zu spalten, um den entstehenden Wasserstoff zusammen mit Kohlendioxid mittels methanogener Mikroorganismen zu Methan umzusetzen. D.h. eine Vorrichtung dieser Art, auch die erfindungsgemäße Vorrichtung, kann durchaus längere Stillzeiten haben, z.B. wenn Strom wegen eines großen Bedarfs nicht zu Gas (hier Methan) umgewandelt wird. Jedoch wäre die in DE 10 2011 051 836 beschriebene Vorrichtung für solche Fälle ungeeignet, da der Biofilm nicht Bestand haben würde, wenn er nicht andauernd mit wässrigem Medium überspült werden würde. Zudem ist er schutzlos einem Eintrag von Sauerstoff ausgesetzt und die methanogenen Mikroorganismen würden sofort absterben.

Die erfindungsgemäße Vorrichtung weist diese Nachteile nicht auf. Zwar beruht der Kern der Erfindung unter anderem auch darauf, die Kontaktoberfläche zwischen den methanogegen Mikroorganismen und dem gasförmigen Gemisch, das Kohlendioxid und Wasserstoff enthält, zu vergrößern, vorzugsweise aber nicht mittels Biofilmbildung und/oder durch Wachstum der methanogenen Mikroorganismen in einem Festbett, Substrat oder Matrix.

Die erfindungsgemäße Vorrichtung weist daher vorzugsweise eine Reaktionssteigerungsvorrichtung zum Vergrößern der Kontaktoberfläche zwischen dem wässrigen Medium mit den methanogenen Mikroorganismen und dem gasförmigen Gemisch, das Wasserstoff und Kohlendioxid enthält, auf, die die Kontaktoberfläche nicht durch Immobilisieren von methanogegen Mikroorgansimen an der Oberfläche von Füllkörpern und/oder nicht durch Berieseln der Füllkörper mit den darauf immobilisierten methanogenen Mikroorganismen und/oder nicht durch Einleiten gasförmiger Substrate über die an der Oberfläche von Füllkörpern immobilisierten methanogenen Mikroorganismen vergrößert.

In den verschiedenen Ausführungsbeispielen der Vorrichtung zum Herstellen vom Methan mittels methanogener Mikroorganismen wirkt die Reaktionssteigerungsvorrichtung derart auf das die methanogenen Mikroorganismen aufweisende wässrige Medium ein, dass seine in Kontakt mit dem Substratgas stehende Oberfläche vergrößert wird. Die Reaktionssteigerungsvorrichtung kann ein Körper sein mit einem sehr großen Verhältnis aus Oberfläche zu Volumen, beispielsweise ein Körper mit einer wabenartigen oder löchrigen Struktur, wobei diese Oberfläche sowohl für das wässrige Medium wie auch für das Substratgas von außen zugänglich ist. Die Vergrößerung der Reaktionsoberfläche mittels der Reaktionssteigerungsvorrichtung kann beispielsweise unter Aufbringung von Energie erfolgen mittels mechanisch dynamischer Mittel wie Düsen, Rührern und dergleichen, mittels mechanisch statischer Mittel wie Sieben oder Durchsickermatrizen, oder aber auch mittels einer Mischung dieser beiden Mittel. Die Reaktionssteigerungsvorrichtung kann eingerichtet sein, eine turbulente Durchmischung oder Verrührung des wässrigen Mediums mit dem Substratgas herbeizuführen. Gleichzeitig oder zusätzlich kann die Reaktionssteigerungsvorrichtung eingerichtet sein, eines der Fluide, d.h. das wässrige Medium oder das Substratgas innerhalb des jeweils anderen Fluids als diskrete Partikel (beispielsweise Gaspartikel oder Flüssigkeitspartikel) zu verteilen, so dass die Kontaktfläche zwischen den beiden Fluiden drastisch vergrößert wird und in erster Näherung der Summe der Oberflächen des zu Partikeln umgewandelten Fluids. Als Beispiele seien hier das Einleiten des Substratgases in das wässrige Medium mittels einer Triebstrahldüse erwähnt, wobei das Substratgas feine Gasblasen (hier als Gaspartikel angesehen) bildet und sich im wässrigen Medium verteilen kann, oder das Zerstäuben oder Dispergieren des wässrigen Mediums (zu kleinen Flüssigkeitspartikeln) in ein Volumen, welches das Substratgas enthält.

Weiterhin ist es eine bevorzugte Ausführungsform der vorliegenden Erfindung, dass die methanogenen Mikroorganismen in dem wässrigen Medium des Reaktors der erfindungsgemäßen Vorrichtung nicht immobilisiert sind. Dies wird z.B. dadurch erreicht, dass dauerhaft mechanische oder pneumatische Energie, z.B. durch Strömung oder Gasblasenbildung in den Reaktor eingebracht wird. Auf diese Weise können die methanogenen Mikroorganismen keinen Biofilm ausbilden. Ebenso ist es vorgesehen, dass den methanogenen Mikroorganismen keine gezielte Möglichkeit zur Bildung eines Biofilms angeboten wird, z.B. durch Einbringen von Oberflächenstrukturen in Form einer Matrix oder für Biofilmbildung geeignete Oberflächenstruktur.

Da die erfindungsgemäße Vorrichtung dazu geeignet ist, Mikroorganismen, insbesondere methanogene Mikroorgansimen aufzunehmen, mit deren Hilfe Methan erzeugt wird, wird die erfindungsgemäße Vorrichtung manchmal hierin auch als "Bioreaktor" bezeichnet.

Der Reaktor der erfindungsgemäßen Vorrichtung ist in einer bevorzugten Ausführungsform als stationärer oder instationärer Reaktor eingerichtet. Der Reaktor ist vorzugsweise ein Feststoffreaktor.

Feststoffreaktoren (englisch Solid State Fermentation-Bioreactors; SFB) sind Reaktoren, die zur Kultur von Mikroorganismen und zur industriellen Herstellung von z.B. Enzymen, Medikamenten und Nahrungsmitteln genutzt werden. Im vorliegenden Fall zur Herstellung von Methan. Bei Feststoffreaktoren wird zwischen stationären und instationären Reaktoren unterschieden. Zu den stationären Reaktoren gehören unter anderem Petrischalen, Fernbach-Kolben, hölzerne Inkubatoren, der Covered Pan-Bioreaktor sowie Säulen-SSF-Bioreaktoren (englisch Solid State Fermentation, SSF, Feststoff-Fermentation). Zu den instationären Reaktoren zählt man die Rotary-Drum-SFB, der gerührte SFB sowie der Taumel-SFB.

Der Reaktor einer erfindungsgemäßen Vorrichtung kann als kontinuierlicher, idealer Rührkessel (CSTR), diskontinuierlicher, idealer Rührkessel (STR), Rohrreaktor, als Schlaufenreaktor, als in Serie geschaltete Reaktoren oder als Kaskade von Reaktoren (Kaskadenreaktor oder Rührkesselkaskade) eingerichtet sein.

In jedem Reaktor finden sich die drei Phasen fest (Biomasse), flüssig (Nährmedium) und gasförmig. Im erfindungsgemäßen Reaktor wird deren Verteilung mit verschiedenen Maßnahmen homogen gehalten, wie z.B. bewegliche mechanische Einbauten (Rührwerke): z. B. im Rührkesselreaktor, äußerer Pumpenkreislauf: die Flüssigkeit wird durch eine Pumpe umgewälzt, z. B. Freistrahlreaktor, Einblasen von Gas: die Gasphase wird in den flüssigen Anteil eingeblasen z. B. Airliftreaktor oder Blasensäulenreaktor. Da der Kern der Erfindung unter anderem darin liegt, dass die methanogenen Mikroorganismen möglichst optimal mit dem gasförmigen Gemisch, enthaltend CO₂ und H₂, in Kontakt gebracht werden ist der Aspekt des Einblasens des gasförmigen Gemischs für die vorliegende Erfindung von besonderer Bedeutung. Daher ist der Airliftreaktor oder Blasensäulenreaktor eine bevorzugte Ausführungsform des Reaktors der erfindungsgemäßen Vorrichtung.

Neben der einfachen Blasensäule werden auch so genannte Schlaufenreaktoren (Blasensäulen mit innerem und äußerem Kreislauf der Flüssigkeit) erfindungsgemäß in einer bevorzugten Ausführungsform eingesetzt. Sie bestehen aus einer Blasensäule, in die koaxial ein Innenrohr eingebracht ist. Das Gas wird über eine Düse oder Fritte so in die Flüssigkeit eingeblasen, dass sich im Innenrohr eine Blasensäule ausbildet. Der Schlaufenreaktor wird in Fällen von flüssigen Reaktionsgemischen hoher Viskosität als Rückvermischungsreaktor eingesetzt. Bei ihm wird die Reaktionsmasse in einem Rohrreaktorsystem durch schlaufenartige Teilrückführungen rückvermischt.

Die Schlaufenreaktoren lassen sich wie folgt unterteilen, wobei alle Unterteilungen bevorzugte Ausführungsformen der vorliegenden Erfindung sind: Strahlschlaufenreaktor und Abstromschlaufenreaktor.

Ein Strahlschlaufenreaktor zeichnet sich durch eine homogene Verteilung von Gas und Flüssigkeit aus. Beim Strahlschlaufenreaktor wird die Flüssigkeit in einem Treibstrahl so am Reaktorboden zugeführt, dass dieser das Gas erfassen und zerteilen kann. Beispiele für Strahlenschlaufenreaktoren sind der Mammut-Schlaufenreaktor oder Strahlschlaufenreaktor. Ein Abstromschlaufenreaktor zeichnet sich durch sehr große Gasverweilzeiten aus, die bei niedriger Bauhöhe eingestellt werden können. Er wird bei der Umsetzung kleinerer spezifischer Gasmengen eingesetzt.

Werden Schlaufenreaktoren mit Leitrohren versehen, dann ergeben sich die folgenden Reaktortypen: Propeller-Schlaufenreaktor (ein Reaktor, bei dem Energie durch ein axial nach unten förderndes Rührorgan eingetragen wird und der mit einem Leitrohr versehen ist), Strahl-Schlaufenreaktor (ein Freistrahlreaktor mit einem Leitrohr), Mammutschlaufenreaktor (ein Airliftreaktor oder ein Blasensäulenreaktor mit einem Leitrohr).

In einer bevorzugten Ausführungsform ist der Reaktor der erfindungsgemäßen Vorrichtung als Submers-Reaktor eingerichtet. In einem erfindungsgemäßen Submers-Reaktor wird die Gas-Flüssigkeits-Austauschfläche durch Dispergierung der Gasphase in der Flüssigkeit aufrechterhalten. Es wird ständig Energie eingetragen: entweder pneumatisch, z.B. Begasung; mechanisch, z.B. durch Rührorgane oder durch Zwangskonvektion der Flüssigkeit, z.B. Flüssigkeitspumpe in einem externen Kreislauf.

Die Ausführungsform als Submers-Reaktor steht im Gegensatz zu der Lehre der DE 10 2011 051 836, die sich gegen Submers-Reaktoren ausspricht und Biofilm-Reaktor bevorzugt. Dabei wurde aber verkannt, dass entgegen der üblichen Betriebsweise von Bioreaktoren möglich ist, Bakterien dauerhaft mit dem H₂ und CO₂ enthaltenden Gasgemisch im wässrigen Medium zu umspülen, ja geradezu mit dem Gasgemisch abzusättigen. Dies wird z.B. dadurch erreicht, dass das Gasgemisch durch Eintrag von mechanischer oder pneumatischer Energie permanent und in großem Ausmaß dem wässrigen Medium zugeführt wird. Die methanogenen Mikroorganismen befinden sich dabei immer in Bewegung und werden permanent von dem Gasgemisch umgeben, so dass sie ausreichend und immer Gelegenheit haben CO₂ und H₂ aufzunehmen und in CH₄ umzusetzen. Normalerweise will man in Bioreaktoren keine Strömungen oder gar eine starke Durchmischung von Gas und wässrigem Medium, um die Fermentation nicht zu stören und den Ertrag dadurch möglicherweise negativ zu beeinflussen. Der Erfinder fand aber heraus, dass methanogene Mikroorganismen stark und permament mit dem H2 und CO2 enthaltenden gasförmigen Gemisch in Kontakt gebracht werden sollen, wobei das gasförmige Gemisch den methanogenen Mikroorgansimen so angeboten wird, dass die Kontaktoberfläche zwischen den methanogenen Mikroorganismen und dem gasförmigen Gemisch möglichst stark vergrößert ist, so dass die methanogenen Mikroorgansimen möglichst optimale Bedingungen zur Aufnahme von CO₂ und H₂ haben. Dies wird beispielsweise dadurch erreicht, dass das gasförmige Gemisch so stark mit dem wässrigen Medium des Reaktors durchmischt wird, z.B. mittels einer Pumpe, vorzugsweise einer Treibstrahlpumpe oder mittels eines Kompressors, dass es zur Schaumbildung kommt.

Daher ist in einer bevorzugten Ausführungsform die Reaktionssteigerungsvorrichtung der erfindungsgemäßen Vorrichtung so ausgestaltet, dass sie das gasförmige Gemisch im wässrigen Medium des Reaktors der erfindungsgemäßen Vorrichtung dispergiert. Die Dispergierung ist dergestalt, dass es beim Einbringen des gasförmigen Gemisches in wässriges Medium des Reaktors der erfindungsgemäßen Vorrichtung zur Schaumbildung kommt.

Der Reaktor der erfindungsgemäßen Vorrichtung ist in einer bevorzugten Ausführungsform als Rohrreaktor eingerichtet. Analog zu Rohr-Photobioreaktoren ist ein erfindungsgemäßer Rohrreaktor aus einem oder mehreren Rohren aufgebaut. Dabei werden Rohre in horizontaler oder vertikaler Ausrichtung aufgebaut, z.B. nebeneinanderliegend.

Wie bereits erwähnt, ist die Reaktionssteigerungsvorrichtung so ausgeführt, dass sie die Kontaktoberfläche zwischen dem wässrigen Medium, in dem sich die methanogenen Mikroorganismen befinden, und dem gasförmigen Gemisch vergrößert. Dies wird bewerkstelligt durch Energieeintrag, z.B. mechanische Energie oder pneumatische Energie. Die Reaktionssteigerungsvorrichtung kann dabei so ausgestaltet sein, dass sie das gasförmige Gemisch im wässrigen Medium des Reaktors der erfindungsgemäßen Vorrichtung dispergiert. Die Dispergierung ist dergestalt, dass es beim Einbringen des gasförmigen Gemisches in wässriges Medium des Reaktors der erfindungsgemäßen Vorrichtung zur Schaumbildung kommt.

In anderen bevorzugten Ausführungsform der erfindungsgemäßen Vorrichtung weist die Reaktionssteigerungsvorrichtung mindestens ein Rieselbett, einen Sprühturm, einen Rohrmischer oder eine Pumpe, vorzugsweise eine Triebstrahlpumpe auf oder einen Kompressor auf. Insbesondere mit Hilfe eines Rohrmischers, einer Pumpe oder eines Kompressors wird gasförmiges Gemisch so in das wässrige Medium eingebracht, dass das gasförmige Gemisch im wässrigen Medium dispergiert wird und es idealerweise zur Schaumbildung kommt, wodurch die Kontaktoberfläche vergrößert wird, so dass die methanogenen Mikroorganismen CO₂ und H₂ effektiv und/oder in größtmöglicher Menge aufnehmen können. Im Fall der vorliegenden Erfindung ist ein Rohrmischer und/oder eine Pumpe, vorzugsweise eine Triebstrahlpumpe mehr bevorzugt.

"Methanogene Mikroorganismen" im Sinne der Erfindung sind solche Mikroorgansimen, die aus Kohlendioxid (CO₂) und Wasserstoff (H₂) Methan (CH₄) herstellen können, d.h. Methanbildner oder methanbildende Mikroorganismen sind. Daher können methanogene Mikroorganismen hierin auch als methanbildende Mikroorganismen bezeichnet werden. Ein Beispiel für solche methanogenen Mikroorgansimen sind methanogene Bakterien, sog. Methanbakterien. Methanbakterien gehören zum Reich der Arachaea und darin zum Stamm Euryarchaeota. Methanogene Mikroorganismen gehören zu den Klassen Methanobacteriales, Methanococcales, Methanomicrobiales, Methanocellales, Methanosarcinales und Methanopyrales. Im Rahmen der vorliegenden Erfindung ist vorgesehen, dass methanogene Mikroorganismen aus allen vorgenannten Klassen stammen können, aber nicht müssen. Es ist daher bevorzugt, dass die methanogenen Mikroorganismen ein Gemisch verschiedener Mikroorganismen des Stamms Euryarchaeota sind.

"Wässriges Medium" umfasst wässrige Flüssigkeiten, bevorzugt solche, in denen methanogene Mikroorganismen wachsen können. Der Fachmann kennt solche Medien. Sie können z.B. Vitamine, Spurenelemente und/oder Schwermetalle, die solche methanogenen Mikroorgansimen benötigen, enthalten. Vorzugsweise ist das wässrige Medium keine ionische Flüssigkeit. Eine ionische Flüssigkeit besteht aus organischen, deren Ionen durch Ladungsdelokalisierung und sterische Effekte die Bildung eines stabilen Kristallgitters behindern. Bereits geringe thermische Energie genügt daher, um die Gitterenergie zu überwinden und die feste Kristallstruktur aufzubrechen. Es handelt sich somit um Salze, die bei Temperaturen unter 100 °C flüssig sind, ohne dass das Salz dabei in einem Lösungsmittel wie Wasser gelöst ist.

In einer bevorzugten Ausführungsform weist die erfindungsgemäße Vorrichtung ferner eine Abführeinrichtung auf, welche zum Abführen eines Gases aus dem mindestens einen Reaktor eingerichtet ist. Das Gas kann H₂, CO₂ und/oder CH₄ enthalten, vorzugsweise enthält das Gas CH₄. Der Stoffmengenanteil von CH4 kann in dem Gas mindestens 50%, 60%, 70%, 80% oder mehr, z.B. 85% oder 90% oder mehr betragen. Der Erfinder hat herausgefunden, dass durch Vergrößern der Kontaktoberfläche zwischen dem wässrigen Medium mit den methanogenen Mikroorganismen und dem gasförmigen Gemisch wie hierin näher erläutert, die Ausbeute an Methan stark erhöht werden kann.

In einer weiteren bevorzugten Ausführungsform weist die erfindungsgemäße Vorrichtung ferner eine Rückführungseinrichtung auf, welche eingerichtet ist zum Rückführen mindestens eines Teils des Gases in die Zuführungseinrichtung. Die Rückführungseinrichtung weist vorzugsweise eine Pumpe oder einen Kompressor auf.

In einer weiteren bevorzugten Ausführungsform weist die erfindungsgemäße Vorrichtung ferner eine Zirkulationseinrichtung auf, die eingerichtet ist zum Zirkulieren des wässrigen Mediums durch den mindestens einen Reaktor. Die Zirkulationseinrichtung weist vorzugsweise mindestens eine Pumpe, vorzugsweise eine Triebstrahlpumpe auf.

In einer anderen bevorzugten Ausführungsform der erfindungsgemäßen Vorrichtung weist die Zuführungseinrichtung eine Vorrichtung zum Anreichern von Kohlendioxid aus einem Gasgemisch auf.

Es ist weiterhin eine bevorzugt, dass die Zuführungseinrichtung der erfindungsgemäßen Vorrichtung ferner eine Einrichtung zum elektrolytischen Spalten von Wasser aufweist.

Ebenso ist es bevorzugt, dass die Zuführungseinrichtung der erfindungsgemäßen Vorrichtung ferner derart eingerichtet ist, dass sie beim Einleiten von H₂ und CO₂ in den mindestens einen Reaktor das gasförmige Gemisch eine turbulente Strömung bildet.

Weiterhin ist es bevorzugt, dass die Zuführungseinrichtung der erfindungsgemäßen Vorrichtung ferner derart eingerichtet ist, dass im Betrieb das Volumen des eingeleiteten H₂ und CO₂ pro Stunde das Fassungsvolumen des mindestens einen Reaktors um mindestens den Faktor 1, 2, 3, 4, 5, 10, 20, 25, 30, 50, 75, 100, 200, 300, 400, 500 oder mehr übersteigt.

Vorzugsweise ist der Druck im Reaktor der erfindungsgemäßen Vorrichtung größer oder gleich 0,1; 0,2; 0,3; 0,4; 0,5; 0,6; 0,7; 0,8; 0,9; 1,0 oder mehr bar.

Vorzugsweise befinden sich die methanogenen Mikroorganismen in Form von makroskopischen Kolonien (Pellets) in dem wässrigen Medium des Reaktors der erfindungsgemäßen Vorrichtung. Als Pellets werden 1-5 mm große Makrokolonien methanogener Mikroorganismen bezeichnet, wie sie in anaeroben Upflowreaktoren (UASB, IC etc.) zur anaeroben Vorreinigung von Industrieabwässern entstehen können. Solche Pellets können aus einer anaeroben Industrieabwasserreinigungsanlage (z.B. Papierfabrik oder Lebensmittelindustrie) erhalten werden. Pellets werden z.B. in Guiot et al. (2011) beschrieben. Es wird angenommen, dass sich die anaeroben methanogenen Mikroorganismen, vermutlich eine Mischpopulation verschiedener methanogener Mikroorganismen, im Inneren eines Pellets befinden, wohingegen sich aerobe Mikroorganismen eher auf der Oberfläche und darunterliegenden Schichten befinden. Die Aerobier schützen sozusagen die Anaerobier vor Sauerstoffzutritt. Im Fall des Beimpfens einer erfindungsgemäßen Vorrichtung, insbesondere des Reaktors, nach dem Austreiben von Sauerstoff, z.B. durch Stickstoff oder mittels einer Kohlenstoffquelle, die von vorhandenen Aerobier unter Verbrauch von Sauerstoff metabolisiert wird, beginnen die methanogenen Mikroorganismen zugeführtes Kohlendioxid und Wasserstoff zu Methan umzusetzen. Der Erfinder hat nun festgestellt, dass solche Pellets aus den vorgenannten Gründen sehr vorteilhaft sind, da beim Arbeiten mit Pellets und beim Beimpfen des Reaktors der erfindungsgemäßen Vorrichtung keine besonderen Vorkehrungen getroffen werden müssen, um anaerobe Bedingung zu schaffen bzw. zu erhalten. Zudem hat der Erfinder festgestellt, dass die Pellets eine so ausreichende Menge methanogener Mikroorganismen enthält, dass innerhalb sehr kurzer Zeit die Herstellung von Methan aus Kohlendioxid und Wasserstoff im Reaktor der erfindungsgemäßen Vorrichtung beginnt. Ferner hat der Erfinder festgestellt, dass ein mit Pellets beimpfter Reaktor einer erfindungsgemäßen Vorrichtung auch nach längerer Stillzeit ohne Weiteres wieder zur Herstellung von Methan angefahren und/oder betrieben werden kann. Aus den genannten Gründen sind Pellets eine bevorzugte Quelle für methanogene Mikroorganismen, die im Rahmen der vorliegenden Erfindung verwendet werden. Ob Pellets, die erfindungsgemäß eingesetzt werden, methanogene Mikroorganismen enthalten, kann einfach mittels z.B. des VIT® Methanogenic bacteria Tests von Vermicon getestet werden. Alternativ dazu können Pellets in einer z.B. miniaturisierten erfindungsgemäßen Vorrichtung, insbesondere in einem Reaktor mit Kohlendioxid und Wasserstoff in einem geeigneten wässrigen Medium begast werden und auf die Herstellung von Methan getestet werden.

Daher betrifft die vorliegende Erfindung in einem weiteren Aspekt die Verwendung von Pellets, enthaltend methanogene Mikroorganismen, zum Beimpfen einer erfindungsgemäßen Vorrichtung.

Wie bereits erwähnt, betrifft die vorliegende Erfindung Verfahren zur Herstellung von Methan in einer Reaktorvorrichtung mittels methanogener Mikroorganismen, die sich in der Reaktorvorrichtung in einem wässrigen Medium befinden, durch Umsetzung von H₂ und CO₂ als Reaktionsgasgemisch, dadurch gekennzeichnet, dass bei der Umsetzung die Kontaktoberfläche zwischen dem wässrigen Medium mit den methanogenen Mikroorganismen und dem gasförmigen Gemisch mittels einer Reaktionssteigerungsvorrichtung vergrößert wird.

Vorzugsweise wird in dem erfindungsgemäßen Verfahren, der Verwendung oder Vorrichtung ein Gemisch von methanogenen Mikroorganismen des Stamms Euryarchaeota eingesetzt. Das Gemisch kommt zustande, da vorzugsweise Pellets eingesetzt werden, die methanogene Mikroorganismen umfassen, und nicht näher charakterisiert sind.

Vorzugsweise werden die methanogenen Mikroorganismn in Form von makroskopischen Kolonien (Pellets) dem wässrigen Medium zugeführt.

Für die Verfahren, aber auch die Vorrichtungen und Verwendungen der vorliegenden Erfindungen kann es von Vorteil sein, wenn das molare Verhältnis von H₂ und CO₂ im gasförmigen Gemisch kleiner oder gleich ungefähr 4 : 1 ist. Es können jedoch auch molare Verhältnisse von größer als 4:1 verwendet werden. Diese können zur Folge haben, dass durch den relativen Mangel an CO₂ der pH-Wert im Inneren des Reaktors ansteigen kann. Durch Zuführung entsprechender Substanzen, beispielsweise Puffer-Systeme oder Säuren, kann der pH-Wert jedoch auf einen gewünschten Wert eingestellt werden.

Die vorliegende Erfindung betrifft auch noch die folgenden Aspekte A1 bis A6:
A1. Verfahren zur Biomethanisierung von H₂ und CO₂ durch methanogene Bakterien bei einem für diese geeignetem Milieu (Temperatur, pH, Redox, Nährstoffe...) in einem Submersbioreaktor, dadurch gekennzeichnet, dass
   1.1. die Umsetzung bei Drücken größer 0,1 oder 1 bar erfolgt,
   1.2. die beteiligten Bakterien dem Reaktor in Form von makroskopischen Kolonien, s.g. Pellets, wie sie in anaeroben Upflowreaktoren (UASB, IC etc.) entstehen können, zugesetzt werden oder in Form pastöser Schlämme aus Faultürmen, Biogasanlagen etc. zugesetzt werden,
   1.3. H₂ und CO₂ dem Reaktor in einem Verhältnis kleiner oder gleich ungefähr 4:1, jedoch auch größer 4:1 zugeführt werden,
   1.4. der Reaktor mit einer Gasrezirkulation durchmischt wird,
   1.5. die Umsetzung in mehreren in Serie geschaltete Reaktorteilen oder mehreren Reaktoren in einer Kaskade erfolgt.
**A2.** Submersbioreaktor nach Aspekt A1, dadurch gekennzeichnet, dass er
   2.1. über ein Flüssigkeitsvolumen (1) und
   2.2. über ein Gasvolumen (2) verfügt.
**A3.** Flüssigkeitsvolumen (1) nach Aspekt 2.1., dadurch gekennzeichnet, dass dieses in einem
   3.1. größeren, begasten, aufwärts durchströmten Teil (A) und
   3.2. einem keineren, unbegasten abwärts strömenden Teil (B) unterteilt ist.
      Alternativ ist das Flüssigkeitsvolumen (1) nach Aspekt 2.1 dadurch gekennzeichnet, dass dieses
   3.1 einen oder mehrere begaste Teil(e) (A) oder
   3.2 in einen oder mehrere begaste Teil(e) (A) und einen oder mehrere unbegaste Teil(e) (B) unterteilt ist.
**A4.** Die interne Gasrezirkulation nach Aspekt 1.4. dadurch gekennzeichnet, dass
   4.1. Gas aus dem gasführenden Volumenteil (2) nach Anspruch 2.2. in den aufströmenden Teil (A) nach Anspruch 3.1. mit geeigneten Mitteln eingebracht wird,
   4.2. die pro Zeitraum rezirkulierte Gasmenge größer ist, als die dem Reaktor in gleichem Zeitraum zugeführte Gasmenge.
      Alternativ ist die interne Gasrezirkulation nach Aspekt 1.4., dadurch gekennzeichnet, dass
   4.1 Gas aus dem gasführenden Volumenteil (2) nach Aspekt 2.2. in die begasten Teil(e) (A) nach Aspekt 3 mit geeigneten Mitteln eingebracht wird.
**A5.** Die Kaskade nach Aspekt 1.5., dadurch gekennzeichnet, dass
   5.1. die Gasrezirkulation nach Anspruch 1.4. für jeden Reaktor oder Reaktorteil getrennt oder für mehre solcher Einheiten gemeinsam erfolgt,
   5.2. sich diese oder Untergruppen dieser sich zusammen in einen Wasserraum (C) befinden und von diesem gemeinsam temperiert werden.
**A6.** Der Submersbioreaktor nach Aspekt 1, dadurch gekennzeichnet, dass dieser über geeignete Vorrichtungen zur Entschäumung verfügt, wie Entschäumerdosierung (6), Schaumzerstörer (7) oder Schaumfallen (7).

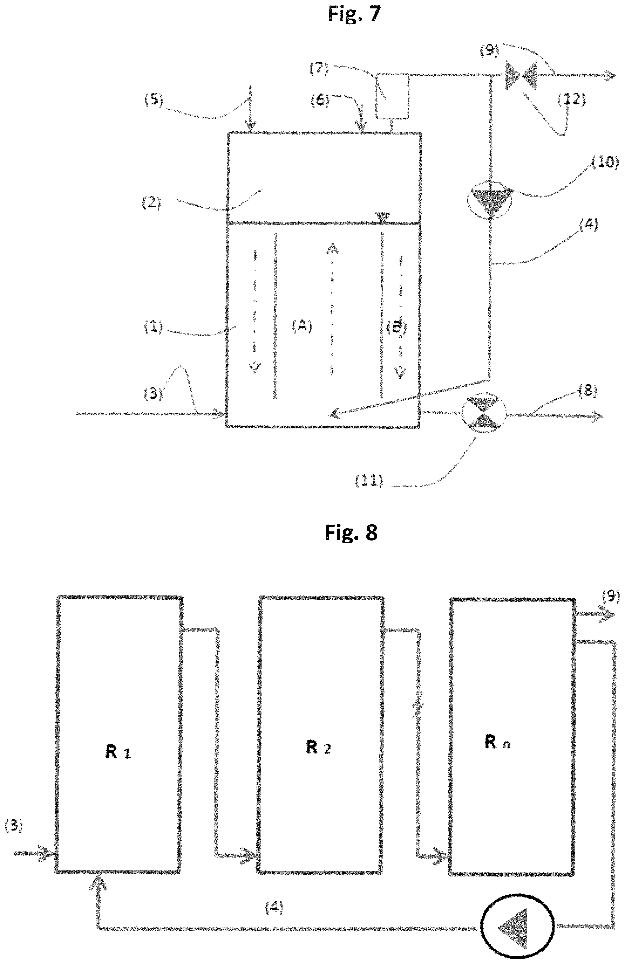

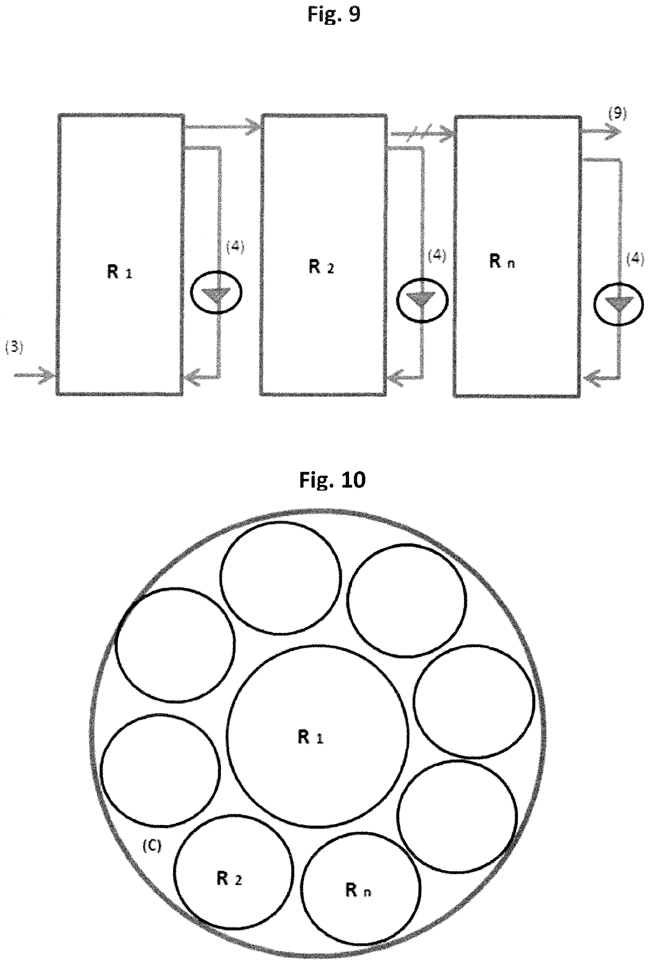

Zu den Bezugszeichen ist auf die in den Figuren 7 bis 10 verwendeten Bezugszeichen zu verweisen, die unterhalb der Legende zu Figur 10 beschrieben werden.

### Nicht-limitierende Beispiele und Abbildungen

Anhand der beigefügten Darstellungen (Figuren) und Ausführungsbeispiele wird die Erfindung näher erläutert.
**Figur 1****:** Fließbild einer beispielhaften Vorrichtung zum Herstellen von Methan mittels methanogener Mikroorganismen unter Verwendung eines Röhrenreaktors
**Figur 2****:** Fließbild einer beispielhaften Vorrichtung zum Herstellen von Methan mittels methanogener Mikroorganismen unter Verwendung eines Rührkessels
**Figur 3****:** Fließbild einer beispielhaften Vorrichtung zum Herstellen von Methan mittels methanogener Mikroorganismen unter Verwendung eines Submersreaktors
**Figur 4****:** Fließbild einer beispielhaften Vorrichtung zum Herstellen von Methan mittels methanogener Mikroorganismen unter Verwendung eines Sprühturms
**Figur 5****:** Fließbild einer beispielhaften Vorrichtung zum Herstellen von Methan mittels methanogener Mikroorganismen unter Verwendung eines Rieselbettreaktors
**Figur 6****:** Fließbild einer beispielhaften Vorrichtung zum Herstellen von Methan mittels methanogener Mikroorganismen unter Verwendung eines Triebstrahlreaktors
**Figur 7****:** Fließbild eines Submers-Reaktors zur Umsetzung gasförmiger Substrate. Beispielsweise werden Methanbakterien als kompakte Pellet in einem Submersbioreaktor eingebracht und unter physiologische Bedingungen mit H₂ und CO₂ unter Druck begast. Ein effektiver Phasenübergang der gasförmigen Substrate wird durch eine interne Rezirkulation (4) von Gasen aus dem Reaktorgasraum (2) in die Flüssigphase (1) bewirkt. Mehrere Reaktoren (Rn) oder Reaktorteile mit unterschiedlichen Bedingungen werden als Kaskade in Serie betrieben. Vorteile des Verfahrens: niedrige Temperaturen, hohe Organismendichte, kurze Anfahrzeiten, flexibler Betrieb, einfache Steuerung.
**Figur 8**: Fließbild einer Kaskade von Reaktoren R₁ bis Rₙ mit gemeinsamer Gasrezirkulation
**Figur 9**: Fließbild einer Kaskade von Reaktoren R₁ bis Rₙ mit getrennter Gasrezirkulation
**Figur 10**: Anordnung mehrerer Reaktoren (R₁ bis Rₙ) in einem gemeinsamen Wasserkörper (C), Querschnitt

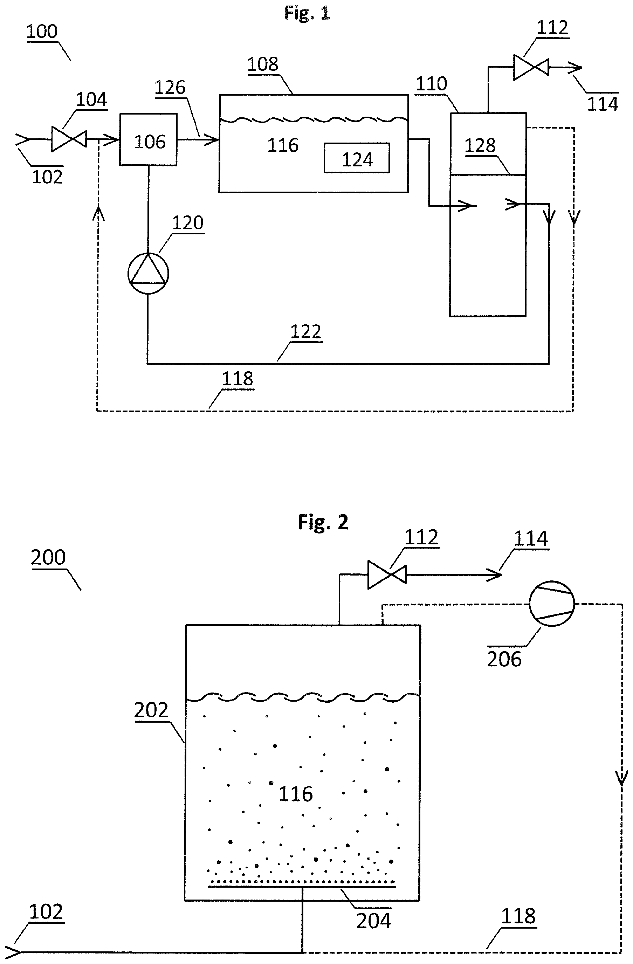

Zeichenliste für die Figuren 1 bis :
- 100: Vorrichtung zum Herstellen von Methan mit einem Rohrreaktor
- 102: Eingangsleitung
- 104: erstes Ventil
- 106: Düse
- 108: Reaktor (als Rohrreaktor eingerichtet)
- 110: Gasabtrennungsvorrichtung
- 112: zweites Ventil
- 114: erster Ausgang
- 116: wässriges Medium
- 118: erster Rückführungszweig
- 120: Pumpe
- 122: zweiter Rückführungszweig
- 124: Mischelement
- 126: Zuführungseinrichtung
- 128: Flüssigkeitsoberfläche
- 200: Vorrichtung zum Herstellen von Methan mit einem Rührkessel
- 202: Reaktor (als Rührkessel eingerichtet)
- 204: Rührwerk
- 300: Vorrichtung zum Herstellen von Methan mit einem Submersreaktor
- 302: Reaktor (als Submersreaktor eingerichtet)
- 400: Vorrichtung zum Herstellen von Methan mit einem Sprühturm
- 402: Reaktor (als Sprühturm eingerichtet)
- 404: Dispersionsvorrichtung
- 500: Vorrichtung zum Herstellen von Methan mit einem Rieselbettreaktor
- 502: Reaktor (als Rieselbettreaktor eingerichtet)
- 504: Matrix
- 600: Vorrichtung zum Herstellen von Methan mit einem Rieselbettreaktor
- 602: Reaktor (als Triebstrahlreaktor eingerichtet)

Zeichenliste für die Figuren 7 bis 10:
- (A): begaster Reaktorteil mit Aufstrom
- (B): unbegaster Reaktorteil mit Abstrom
- (C): Wasserkörper, über den mehrere Reaktoren temperiert werden können
- (1): überwiegend mit Wasser gefüllter Volumenanteil des Reaktors
- (2): überwiegend mit Gas gefüllter Volumenanteil des Reaktors
- (3): Gaszuführung
- (4): Gasrezirkulation
- (5): Zugang zur Nährstoff- und Bakterienzufuhr, pH-Einstellung, Probenahme etc.
- (6): Entschäumerdosierung
- (7): Entschäumungsvorrichtung, Schaumfalle
- (8): Abführung fester, flüssiger und gelöster Produkte aus (1)
- (9): Abführung gasförmiger Produkte aus (2)
- (10): Gasrezirkulationspumpe
- (11): Absperrorgan
- (12): Druckhalteventil

### Beschreibung konkreter Ausführungsbeispiele der Erfindung

In **Fig.1** ist eine beispielhafte Ausführungsform einer Vorrichtung 100 zum Herstellen von Methan mittels methanogener Mikroorganismen durch Umsetzung von Wasserstoff und Kohlenstoffdioxid dargestellt. Die beispielhafte Vorrichtung 100 weist einen Reaktor 108 auf. Eine Eingangsleitung 102 ist über ein erstes Ventil 104, beispielsweise ein erstes Druckhalteventil 104, an einen ersten Eingang einer Düse 106 gekoppelt, beispielsweise einer Triebstrahldüse. Die Düse 106 ist über eine entsprechende Leitung mit dem Reaktor 108 gekoppelt. Der Reaktor 108 ist an seinem Ende mittels einer Leitung mit einem Eingang einer Gasabtrennungsvorrichtung 110 gekoppelt. Die Gasabtrennungsvorrichtung 110 kann mit einer Flüssigkeit, beispielsweise Wasser, gefüllt sein und weist drei Ausgänge auf. An einem ersten Ausgang 114 kann über ein zweites Ventil 112, beispielsweise ein zweites Druckhalteventil, eine gasförmige Phase abgeführt werden, welche zumindest das Endprodukt der mikrobiellen Umwandlung, das Methangas, aufweist oder im Wesentlichen aus diesem besteht. Über einen zweiten Ausgang kann optional ein Teil derselben gasförmigen Phase aus der Gasabtrennungsvorrichtung abgeführt werden und über einen ersten Rückführungszweig 118 der Düse 106 zugeführt werden. Die gasförmige Phase kann dabei mittels einer separaten Leitung in die Düse 106 eingespeist werden oder sie kann in die Leitung eingespeist werden, mittels welcher das erste Ventil 104 mit der Düse 106 gekoppelt ist. Bei dem ersten Rückführungszweig 118 handelt es sich um eine optionale Vorrichtung, insbesondere bei ausreichend langen Rohrreaktoren, welche für die Funktion der hier vorgestellten Erfindung nicht wesentlich ist, jedoch durchaus zur Optimierung ihres Betriebes geeignet sein kann. An einem dritten Ausgang der Gasabtrennungsvorrichtung 110 kann eine flüssige Phase abgeführt werden und mittels eines zweiten Rückführungszweiges 122, in welchem eine Pumpe 120 vorgesehen ist, der Düse 106 zugeführt werden.

In der in Fig.1 dargestellten Ausführungsform handelt es sich bei dem Reaktor 108 um einen Rohrreaktor. Unter einem Rohrreaktor kann hierbei ein röhrenförmiger Behälter zu verstehen sein, in dem die methanogenen Mikroorganismen enthalten sind, und in dem eine mikrobielle Umwandlungsreaktion abläuft, bei der aus H₂ und CO₂ Methan hergestellt wird. Das H₂ und CO₂ bilden zusammen das Substratgas - das Ausgangsprodukt der mikrobiellen Umwandlungsreaktion. Diese beiden Gase können getrennt voneinander mittels separater Leitungen oder bereits als Gasgemisch mittels einer Leitung der Düse 106 zugeführt werden. Letzterer Fall ist in der in Fig.1 gezeigten Ausführungsform verwirklicht .Der Reaktor 108 kann derart eingerichtet sein, dass sein Inneres auf einer bestimmten Temperatur gehalten werden kann, welche für einen optimalen Ablauf der Reaktion sorgt. Im vorliegenden Fall der Erzeugung von Methan mittels methanogener Mikroorganismen kann die Temperatur beispielsweise in einem Bereich von ungefähr 30°C bis ungefähr 80°C liegen, beispielsweise in einem Bereich von ungefähr 40°C bis ungefähr 70°C, beispielsweise bei ungefähr 40 bis ungefähr 50°C. In dem Reaktor 108 ist ein wässriges Medium 116 enthalten, wobei sich die methanogenen Mikroorganismen in dem wässrigen Medium 116 befinden. Bei dem wässrigen Medium 116 kann es sich um ein Gas/Flüssigkeitsgemisch handeln, beispielsweise ein schaumiges Gas/Wassergemisch. Das Substratgas, welches H₂ und CO₂ aufweist, wird mittels der Düse 106 über eine Zuführungseinrichtung 126 dem Reaktor 108 zugeführt. Bei der Zuführungseinrichtung 126 kann es sich um ein entsprechend eingerichtetes Rohrsystem handeln, welches zum Einleiten eines Fluids in den Reaktor 108 eingerichtet ist. Im Rahmen dieser Beschreibung kann unter einem Fluid ein Gas, eine Flüssigkeit oder eine Mischung draus verstanden werden, etwa eine fein zerstäubte Flüssigkeit, welche zudem mit einem Gas vermengt ist. Selbstverständlich können noch weitere Substanzen, wie etwa die mikrobielle Umwandlungsreaktion begünstigende Additive wie etwa Schwermetalle in geringen Mengen, die von methanogenen Mikroorganismen zum Teil benötigt werden, in den Reaktor 108 geleitet werden.

Mittels des ersten Rückführungszweigs 118 kann die gasförmige Phase aus der Gasabtrennungsvorrichtung 110 der Düse 106 zugeführt werden und so wiederverwertet werden. Bei der mittels des ersten Rückführungszweigs 118 abgeführten gasförmigen Phase kann es sich überwiegend um Bestandteile des Substratgases handeln, welche im Reaktor 108 nicht zu Methan umgesetzt worden sind. Diese nicht umgesetzten "Reste" können auf diesem Wege sozusagen recycled werden und stehen der mikrobiellen Reaktion erneut zur Verfügung. Der erste Rückführungszweig 118 kann bei sehr langen Rohrreaktoren 108 entfallen, da das am Eingang eines solchen Rohrreaktors 108 eingeleitete Substratgas während bis zum Ende des Reaktors 108 mit großer Wahrscheinlichkeit umgewandelt wird. Anders ausgedrückt kann davon ausgegangen werden, dass mit zunehmender Länge des Rohrreaktors 108 der Anteil des am Ende eines solchen Reaktors nicht umgesetzten Substratgases immer kleiner wird.

Die zwischen dem ersten Ventil 104 und dem Reaktor 108 angeordnete Düse 106 dient zum Einleiten zumindest des Substratgases in den Reaktor 108 derart, dass darin zugleich eine Umwälzung oder Durchsetzung des flüssigen Mediums 116 mit dem so eingeleiteten Substratgas erfolgt. Mittels der Düse 106 kann kinetische Energie des Substratgases erhöht werden, bevor es in den Reaktor 108 eingeleitet wird. Dadurch kann erreicht werden, dass das eingeleitete Substratgas sozusagen in das wässrige, die methanogenen Mikroorganismen aufweisende Medium eingeblasen wird, wodurch eine starke Umwälzung des wässrigen Mediums 116 erfolgt. Anders ausgedrückt kann durch das Einleiten des Substratgases in den Reaktor 108 mittels der Düse 106 erreicht werden, dass sich zahlreiche Gasblasen bilden, welche die wässrige Phase 116 durchsetzen (im Folgenden als dynamischer Fall bezeichnet). Dadurch kann die Reaktionsoberfläche zwischen dem Substratgas und dem wässrigen Medium 116 drastisch vergrößert werden im Vergleich zu dem Fall (im Folgenden als statischer Fall bezeichnet), in dem das Substratgas in einen Rohrreaktor 108 eingeleitet wird, ohne dass die eben beschriebene Umwälzung der wässrigen Phase 116 erfolgt.

Der Triebstrahl kann von der Düse 106 mit Hilfe der flüssigen Phase gebildet werden, welche aus der Gasabtrennungsvorrichtung 110 abgeführt wird. Bei der Düse 106 kann es sich beispielsweise um eine Strahlpumpe handeln, bei welcher je nach Ausführungsform der Vorrichtung beispielsweise das abgeführte gasförmige Medium oder die flüssige Phase aus der Gasabtrennungsvorrichtung 110 verwendet werden kann. Bei der über den zweiten Rückführungszweig 122 abgeführten flüssigen Phase handelt es sich um das wässrige Medium 116 aus dem Reaktor 108. Die Gasabtrennungsvorrichtung 110 kann funktional als Beruhigungs- oder Entmischungsvorrichtung gesehen werden. Im Reaktor 108 kann auf Grund der turbulenten Mischvorgänge das wässrige Medium 116 als ein schaumiges Gas/Wassergemisch vorliegen. Anders ausgedrückt ist die die methanogenen Mikroorganismen enthaltende Flüssigkeit stark mit der gasförmigen Phase - Methan und Resten des Substratgases - angereichert. In diesem Zustand wird das wässrige Medium 116 in die Gasabtrennungsvorrichtung 110 übergeleitet und dort erfolgt eine Abtrennung der gasförmigen Phase von der flüssigen Phase, wobei dort das wässrige Medium 116 immer noch schaumig sein kann. Dieser Gasabtrennungsprozess kann ganz natürlich durch Aufsteigen der gasförmigen Phase (nicht umgewandelte Reste des Substratgases und Methan) aus dem Gemisch 116 von Flüssigkeit und Gas erfolgen. Die von der gasförmigen Phase im Wesentlichen bereinigte Flüssigkeit mitsamt der methanogenen Mikroorganismen kann dann, wie aus Fig.1 ersichtlich, am dritten Ausgang der Gasabtrennungsvorrichtung 110 aus dieser entnommen und mittels der Pumpe 120 über den zweiten Rückführungszweig 122, der Düse 106 zugeführt und zur Bildung des in den Reaktor 108 eintretenden Treibstrahls verwendet werden. Auf diese Weise kann ein geschlossener Kreislauf des wässrigen Mediums 116 realisiert werden.

Das am zweiten Ausgang der Gasabtrennungsvorrichtung 110 abführbare Gas, welches mittels des optionalen ersten Rückführungszweigs 118 der Düse 106 zugeführt werden kann, weist im Optimalfall im Wesentlichen nur Methan auf, ggfs. geringe Reste des nicht umgewandelten Substratgases. Die Rezirkulationsrate der gasförmigen Phase aus der Gasabtrennungsvorrichtung 110 kann an die Länge des Reaktors 108 angepasst werden und, wie bereits erwähnt, bei ausreichend langen Reaktoren 108, bei welchen die rezirkulierte gasförmige Phase aus der Gasabtrennungsvorrichtung 110 im Wesentlichen aus Methan besteht, gegen Null gehen. Da das hier vorgestellte Herstellungsverfahren sehr effizient abläuft, d.h. durch die starke Durchmischung des wässrigen Mediums 116 im Reaktor 108 mit dem Substratgas, können Umwandlungsraten von beispielsweise mehr als 90%, beispielsweise mehr als 95%, beispielsweise nahezu 100% erreicht werden. Bei vorhandenem ersten Rezirkulationszweig 118 kann die Rezirkulationsrate der gasförmigen Phase aus der Gasabtrennungsvorrichtung 110 stets größer ausfallen als die Zuführungsrate des Substratgases in den Reaktor 108 mittels der Eingangsleitung 102, beispielsweise um Faktoren in der Größenordnung von etwa 1 bis beispielsweise 100, bis beispielsweise 200, bis beispielsweise 500, bis beispielsweise 1000 oder größer. Bei sehr langen Rohrreaktoren 108, wie oben erwähnt, wird nahezu das gesamte Substratgas zu Methan umgesetzt. Ein entsprechend dafür ausgelegter Rückführungszweig 118 kann auch in diesem Fall trotz verschwindend geringem Anteil der Substratgasrestes am rezirkulierten Gas bereitgestellt werden. Unabhängig von der Umwandlungsrate innerhalb des Reaktors 108 kann mit Hilfe einer hohen Gasrezirkulationsrate mittels des ersten Rückführungszweigs 118, welcher damit funktional einem Rezirkulationszweig entspricht, der Gasanteil im Gas-Wassergemisch im Inneren des Reaktors 108 und damit der Grad der Turbulenz erhöht werden. Mittels der Gasrezirkulation kann die Durchmischung des Gas-Wassergemisches innerhalb des Rektors 108, also beispielsweise die Kontaktzeit und Kontaktfläche zwischen den biesen beiden Phasen, erhöht werden.

Das sich über der Flüssigkeitsoberfläche 128 in der Gasabtrennungsvorrichtung 110 befindende Gasgemisch weist im Betrieb der Vorrichtung 100 das Produktgas und je nach Umwandlungseffizienz kleine bis verschwindend geringe Reste von nicht umgewandeltem Substratgas auf. Dieses Gasgemisch kann gänzlich oder teilweise am ersten Ausgang 114 aus der Vorrichtung 100 abgeführt werden und optional teilweise mittels des ersten Rückführungszweigs 118 in den Reaktor 108 rezirkuliert werden. Anders ausgedrückt wird an dem ersten Ausgang 112 und am zweiten Ausgang (zugehörig zum optionalen ersten Rückführungszweig 118) eine gasförmige Phase gleicher Zusammensetzung abgeführt.

Generell liegt bei einem bestimmten H₂/CO₂ Verhältnis im Reaktor 108 bzw. in der Gasabtrennungsvorrichtung 110 ein Gleichgewicht vor: einer CO₂-Konzentarion im Gas entspricht eine CO₂-Konzentration im wässrigen Medium 116. Ist das gesamte CO₂ umgesetzt, steigt der pH-Wert im wässrigen Medium 116, was unerwünscht sein kann. Daher kann die Zuführung des Substratgases an der Eingangsleitung 102 so eingestellt werden, dass sowohl in der am ersten Ausgang 114 und damit auch am zweiten Ausgang abgeführten Gasphase immer ein Rest an CO₂ übrig bleibt, beispielsweise etwa 1% bis etwa 2%. Eine vollständige Umsetzung von H₂ hingegen führt nicht zu einer Verschiebung des pH-Wertes. Zur Stabilisierung des pH-Wertes kann es somit von Vorteil sein, wenn der Vorrichtung 100 mittels der Eingangsleitung 102 das Substratgas im Verhältnis von etwa 4:1 (H₂ zu CO₂) oder weniger zugeführt wird.

Das Einleiten des Substratgases in den Reaktor 108 mittels der Düse 106, welche zur Durchmischung des wässrigen Mediums 116 mit dem Substratgas führt, sorgt dafür, dass pro Zeiteinheit im dynamischen Fall eine weitaus größere Anzahl der methanogen Organismen in Kontakt mit dem Substratgas gelangt und daher eine größere Anzahl der methanogen Organismen pro Zeiteinheit Methan produzieren kann als im statischen Fall. Näherungsweise entspricht die Reaktions- bzw. Kontaktoberfläche zwischen einem sich im Reaktor befindlichen wässrigen Medium 116 und dem Substratgas im statischen Fall der Oberfläche des Mediums. Im dynamischen Fall hingegen, also gemäß der in dieser Beschreibung geschilderten erfindungsgemäßen Lehre, ist diese Fläche wesentlich größer, da zu der ohnehin schon aufgerauten und damit größeren Oberfläche des wässrigen Mediums 116 im Reaktor 108 (angedeutet durch die gewellte Linie innerhalb des Reaktors 108) die Summe der Oberflächen der Gasblasen hinzugerechnet werden muss, welche das wässrige Medium 116 durchsetzen, wobei tendenziell am Anfang des Rohrreaktors 108 die Gasblasen ausschließlich Substratgas aufweisen können, zu dessen Ende hin die Gasblasen idealerweise fast ausschließlich das Endprodukt Methan aufweisen können und dazwischen das die Blasen bildende Gas ein beliebiges Mischungsverhältnis aus Substratgas und Methan aufweisen kann.

Zur weiteren Vergrößerung der Kontaktoberfläche zwischen dem Substratgas und dem wässrigen Medium 116 kann der Reaktor 108 ferner optional mindestens ein Mischelement 124 aufweisen. Bei dem mindestens einen Mischelement 124 kann es sich um mindestens ein Elemente handeln, welches geeignet ist das sich im Reaktor 108 befindliche Substratgas mit dem wässrigen Medium 116 zu vermischen. Das mindestens eine Mischelement 124 kann beispielsweise ein mechanisch dynamisches Element sein und beispielsweise als ein sich drehendes Wasserrad, ein Leitstrahlmischer, ein Turbinenrührer, ein Propellerrührer oder etwa ein Rohrmischer eingerichtet sein, wobei dies keineswegs als eine abschließende Aufzählung möglicher Mischelemente gesehen werden soll. Das mindestens eine Mischelement 124 kann aber auch ein mechanisch statisches Element sein, beispielsweise ein Zerstäubungssieb, welches größere Substratgasblasen in kleinere Substratgasblasen umwandelt. Das mindestens eine Mischelement 124 kann auch sowohl mindestens ein mechanisch dynamisches und ein mechanisch statisches Element aufweisen.

Wie ferner in Fig.1 dargestellt, weist die beispielhafte Vorrichtung 100 zum Herstellen von Methan mittels methanogener Mikroorganismen mindestens zwei Ventile auf, nämlich das erste Ventil 104 und das zweite Ventil 112. Es können klarerweise nach Bedarf weitere strömungstechnische Elemente in der Vorrichtung 100 vorgesehen sein wie weitere Ventile, Durchflusszähler und Verdichter. Mittels des ersten Ventils 104 und des zweiten Ventils 112 kann der Teil der beispielhaften Vorrichtung 100 zwischen dem das Substratgas zuführenden Eingangsleitung 102 und dem die gasförmige, Methangas aufweisende Phase abführenden, ersten Ausgang 114 der Gasabtrennungsvorrichtung 110 einem Druckgefälle ausgesetzt werden, welches das Beschicken des Reaktors 108 ermöglicht und eine Fluidströmung vom Reaktor 108 zur Gasabtrennungsvorrichtung 110 herbeiführt. Das untere Druckniveau (d.h. der Druck am zweiten Ventil 112) des Druckgefälles kann bei Umgebungsdruck (Atmosphärendruck) liegen. Bei Bedarf kann das untere Druckniveau jedoch auch bei einem höheren Druck als dem Umgebungsdruck liegen, beispielsweise ungefähr 50 mbar, beispielsweise 100 mbar, beispielsweise 200 mbar, beispielsweise 500 mbar oder mehr über dem Umgebungsdruck. Dieses kann sich als vorteilhaft erweisen, da so effektiv verhindert werden kann, dass Sauerstoff in die Vorrichtung 100 eindringen kann, welcher sich üblicherweise negativ auf die Lebensdauer und/oder Anzahl der methanogenen Mikroorganismen auswirken kann. Das erste Ventil 104 kann zudem auch dafür verwendet werden, die Menge des dem Reaktor 108 zugeführten Substratgases pro Zeiteinheit und dessen Mischungsverhältnis zu steuern. Letzteres kann auch durch Bereitstellen von getrennten Zuführungen für H₂ und CO₂ und von entsprechenden zwei ersten Ventilen eingestellt werden.

Die effektive Umwandlung des Substratgases mittels der methanogenen Mikroorganismen zum Produktgas Methan beruht unter anderem auf einer hochturbulenten Gas-Wasser-Bakterien-Durchmischung innerhalb des Rohrreaktors 108 bei einer ausreichendenden Durchflussgeschwindigkeit des wässrigen Mediums 116, welche bei Verwendung von statischen Rohrmischern beispielsweise im Bereich von etwa 3 m/s bis etwa 10 m/s liegen kann, beispielsweise im Bereich von etwa 3 m/s bis etwa 5 m/s. Bei Verwendung von dynamischen Rohrmischern mit selbstständigem Motorantrieb ist eine Durchflussgeschwindigkeit nicht vorgegeben.

Die gesteigerte Reaktionsrate innerhalb des Reaktors 108 kann durch eine hochturbulente Durchmischung des wässrigen Mediums 116 mit dem Substratgas erreicht werden. Vorzugsweise kann das pro Stunde in den Reaktor 108 eingeleitete Gasvolumen über dem Fassungsvolumen des Rohrreaktors 108 selbst liegen. Das Verhältnis des pro Stunde in den Reaktor 108 eingebrachten Gasvolumens zum Fassungsvolumen desselben kann beispielsweise circa 5:1 oder mehr, beispielsweise circa 10:1, beispielsweise circa 25:1, beispielsweise 50:1 oder mehr betragen. Diese Werte beziehen sich auf die Gaseinleitung an der Zuführungseinrichtung 102.

Auf diesen Zahlen basierend soll hier eine kleine Überschlagsrechnung zur Demonstration der möglichen Leistungsfähigkeit des hier vorgestellten Konzepts erfolgen. Geht man von einem Verhältnis des pro Stunde in den Reaktor 108 eingebrachten Gasvolumens zum Fassungsvolumen desselben von 5:1 aus, kann pro Kubikmeter Reaktorvolumen bei einer gemäß der Lehre dieser Beschreibung realisierbaren nahezu vollständigen Umsetzung des Substratgases pro Stunde 1 m³ Methan gebildet werden. 1 m³ Methan hat einen Energiegehalt von ca. 10 kWh. Aus der bei vollständiger Umwandlung der Substratgase zu Methan die Entwicklung von 1 m³ Methan pro Stunde auf 1 m³ Reaktorvolumen folgt, dass damit ein entsprechender Reaktor pro Kubikmeter eine Leistung von ca.10 kW aufweist. Ein 100 m³ großer Reaktor hätte also eine Leistung von 1 MW. Steigert man das Gasdurchsatzverhältnis auf 25:1 pro Stunde, so ließe sich bei vollständiger Umwandlung der Substratgase zu Methan eine Reaktoranlage mit 1 MW Leistung mit nur 20 m³ Reaktorvolumen realisieren. Hier ist jedoch zu betonen, dass es sich hierbei um eine beispielhafte Rechnung handelt, welche ein mögliches Betriebsszenario der Vorrichtung zum Herstellen von Methan mittels methanogener Mikroorganismen überschlagsmäßig quantitativ veranschaulichen soll und nicht den Leistungsspielraum des hier vorgestellten Konzepts in irgendeiner Weise eingrenzt.

Die hochturbulente Durchmischung des wässrigen Mediums 116 mit dem Substratgas innerhalb des Reaktors 116 - durch welche Mittel sie auch herbeigeführt sein mag - sorgt für eine deutliche Vergrößerung der Kontaktoberfläche zwischen dem wässrigen Medium 116 mit den methanogenen Mikroorganismen und dem Substratgas, was wiederum für eine deutlich vergrößerte Reaktionsrate innerhalb des Reaktors 108 sorgt im Vergleich zum weiter oben erwähnten statischen Fall. In Anbetracht der starken Durchmischung des wässrigen Mediums 116 kann vorliegend von einem eher untypischen Betrieb des Rohrrektors 108 der beispielhaften Vorrichtung 100 zum Herstellen von Methan mittels methanogener Mikroorganismen gesprochen werden, da artgleiche in der Chemie verwendete Rohrreaktoren üblicherweise im Betrieb eine vernachlässigbare axiale Durchmischung aufweisen. Bei dem hier vorgestellten Herstellungsverfahren von Methan mittels der Vorrichtung 100 aus Fig.1 als einem Ausführungsbeispiel ist es jedoch unter anderem diese Durchmischung, welche für eine gesteigerte Reaktionsrate innerhalb des Reaktors sorgt und damit für eine deutlich gesteigerte Methanausbeute im Vergleich zu üblichen Herstellungsbedingungen. In diesem Zusammenhang kann die Düse 106 in Fig.1 von der Funktion her als eine Reaktionssteigerungsvorrichtung gesehen werden, da der von ihr erzeugte Triebstrahl im Reaktor 108 eine Umwälzung des wässrigen Mediums 116 hervorruft und so durch die besagte Vergrößerung der Kontaktoberfläche die erhöhte Reaktionsrate erreicht werden kann. Auch das optional im Reaktor 108 bereitgestellte Mischelement 124 trägt zur Umwälzung des wässrigen Mediums 116 innerhalb des Rohrreaktors 108 bei und ist damit funktional ebenfalls der Reaktionssteigerungsvorrichtung zuzuordnen.

Die in Fig.1 dargestellte und bisher beschriebene beispielhafte Ausführungsform der Vorrichtung 100 zum Herstellen von Methan verkörpert eine Möglichkeit, wie gemäß der hier vorgestellten Lehre eine hocheffiziente Umsetzung des Substratgases zu Methan erfolgen kann. Nachfolgend werden auf Basis der Vorrichtung aus Fig.1 weitere beispielhafte Ausführungsformen beschrieben. Dabei sind ausgehend von Fig.1 funktional gleiche Elemente mit gleichen Bezugszeichen versehen und werden nicht erneut im größeren Detail beschrieben.

Eine weitere beispielhafte Ausführungsform einer Vorrichtung 200 zum Herstellen von Methan mittels methanogener Mikroorganismen ist in **Fig.2** dargestellt. Die Vorrichtung 200 basiert im Unterschied zu der in Fig.1 dargestellten Ausführungsform auf einem Rührkessel 202 (engl: STR, stirred tank reactor bzw. CSTR, continuous stirred tank reactor), in welchem sich das wässrige Medium 116 befindet, wobei der Rührkessel 202 zur Kategorie der Submersreaktoren zuzuordnen ist. Der Rührkessel 202 weist ein Rührwerk 204 auf, welches funktional als Mischelement fungiert. In dem Rührwerk 204, beispielsweise auf der Oberfläche seiner Rotorblätter, können Öffnungen vorgesehen sein, aus denen Gas ausströmen kann. Wie bei der in Fig.1 gezeigten Vorrichtung 100 wird auch hier das Substratgas mittels der Eingangsleitung 102 dem Reaktor zugeführt. Das Substratgas kann dann, wie in Fig.2 gezeigt, aus dem Rührwerk 204 selbst, beispielsweise aus seinen Rotorblättern, während dessen Drehung (Rührbewegung) austreten oder alternativ mittels einer gesonderten, vom Rührwerk 204 unabhängigen Einleitungseinrichtung in das wässrige Medium 116 eingebracht werden. Eine solche Einleitungseinrichtung kann beispielsweise am Boden des Rührkessels 202 angeordnet sein und über eine Anzahl von Öffnungen das Substratgas in das wässrige Medium 116 einleiten. In beiden Fällen wird eine optimale Vermengung des Substratgases mit dem wässrigen Medium 116 erreicht. Durch die Rotation des Rührwerks 204 oder durch das Zusammensiel des Rührwerks 204 mit der darunter angeordneten Einleitungseinrichtung (nicht in Fig.2 gezeigt) kann eine optimale Vermischung des Substratgases mit dem wässrigen Medium 116 erreicht werden, verdeutlicht durch die in Fig.2 dargestellten, im wässrigen Medium 116 aufsteigenden Gasblasen. Eine starke Gasblasenbildung kann erwünscht sein, denn dadurch kann die Reaktionsoberfläche zwischen dem in den Rührkessel 202 eingeleiteten Substratgas und dem wässrigen Medium 116 deutlich vergrößert werden. Unter diesem Aspekt kann die Bildung vieler kleiner Gasblasen besser sein als die Bildung weniger größerer Gasblasen. Weiter kann eine hohe Gasrezirkulation von Vorteil sein, welche eine gesteigerte Kontaktzeit und Kontaktoberfläche zwischen dem wässrigen Medium und dem Substratgas bewirken kann. Die methanogenen Mikroorganismen im wässrigen Medium 116 können - zusätzlich zur Versorgung an der Grenzschicht zwischen wässrigem Medium 116 und der darüber liegenden Gasphase innerhalb des Rührkessels 202 - über die Oberfläche einer jeden Gasblase mit den Produktgasen H₂ und CO₂ versorgt werden, so dass in erster Näherung das gesamte Volumen des flüssigen Mediums 116 zur Bildung von Methan verwendet kann. Um die Bildung kleiner Gasblasen zu fördern, kann das Rührwerk 204 entsprechende Elemente wie Siebchen oder andere feinmaschige Strukturen auf seiner Oberfläche aufweisen.

Mittels des ersten Rückführungszweigs 118 kann die gasförmige Phase, mitunter ggfs. nicht umgewandeltes Substratgas enthaltend, welche sich über der Oberfläche des wässrigen Mediums 116 im Inneren des Rührkessels 202 ansammelt, abgeführt werden und über einen Verdichter 206 dem an der Eingangsleitung 102 zugeführten Substratgas beigemengt werden und so für eine gesteigerte, turbulente Durchmischung der Phasen im Inneren des Rührkessels 202 sorgen.

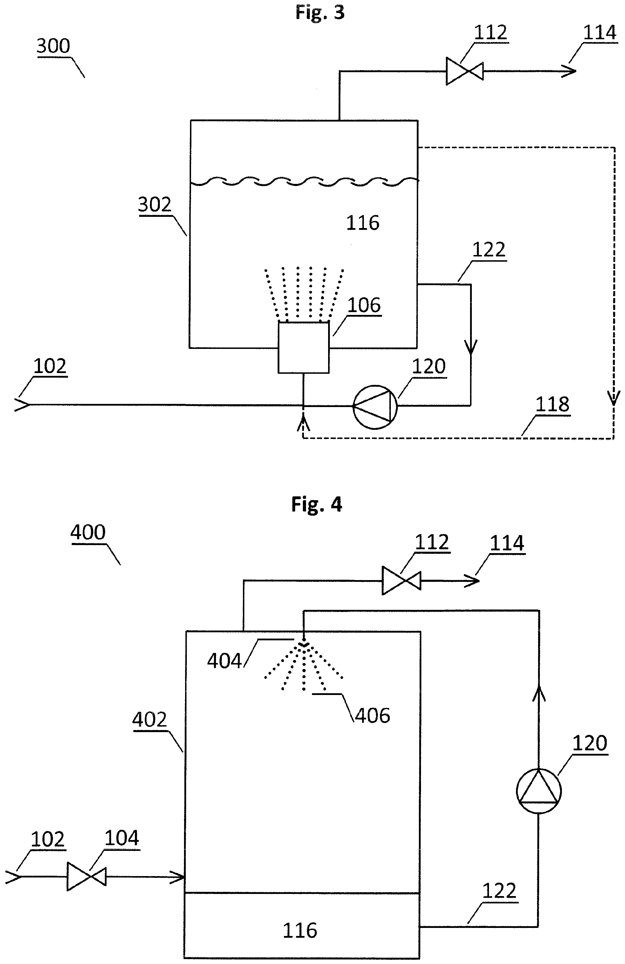

Eine weitere beispielhafte Ausführungsform einer Vorrichtung 300 zum Herstellen von Methan mittels methanogener Mikroorganismen ist in **Fig.3** dargestellt. Die Vorrichtung 300 basiert auf einem Submersreaktor 302. Am Boden des Submersreaktors 302 ist die Düse 106 angeordnet, beispielsweise eine Triebstrahldüse, mittels welcher ein Triebstrahl erzeugt und vom Boden des Submersreaktors 302 in das wässrige Medium 116 hinein eingestrahlt werden kann. Mit der Düse 106 kann unter Energieeintrag zum einen eine Zwangskonvektion des wässrigen Mediums 116 erreicht werden, zum anderen kann das wässrige Medium 116 mit dem Substratgas turbulent vermengt werden. Funktional gesehen entspricht die Düse 106 damit der Reaktionssteigerungsvorrichtung, wobei in dem Submersreaktor 302 selbstverständlich zusätzlich mindestens ein Mischelement 124 vorgesehen sein kann, welches in Zusammenhang mit der in Fig.1 gezeigten Vorrichtung weiter oben beschrieben worden ist. Die Düse 106 wird in gleicher Weise wie bereits in Zusammenhang mit der in Fig.1 gezeigten Vorrichtung 100 mit Fluiden versorgt, nämlich mit dem Substratgas, mit dem wässrigen Medium 116 über den zweiten Rückführungszweig 122 und optional mit der gasförmigen Phase aus dem Inneren des Submersreaktors 302.

Eine noch weitere beispielhafte Ausführungsform einer Vorrichtung 400 zum Herstellen von Methan mittels methanogener Mikroorganismen ist in **Fig.4** dargestellt, bei welcher als Reaktor ein Sprühturm 402 verwendet wird. Das Substratgas wird mittels des ersten Ventils 104 in den Innenraum des Sprühturms 402 eingeleitet. Mittels des zweiten Rückführungszweigs 122 wird das wässrige Medium 116 nach oben zu einer Dispersionsvorrichtung 404, beispielsweise einer Zerstäubungsdüse, transportiert. Bei diesem Ausführungsbeispiel wird das wässrige Medium 116 zu feinsten Tröpfchen 406 zerstäubt und an der Decke des Sprühturms 402 in diesen eingesprüht. Die Dispersionsvorrichtung 404 ist damit funktional gesehen eine Reaktionssteigerungsvorrichtung. Je feiner die von der Dispersionsvorrichtung 404 erzeugten Tröpfchen 406, desto größer die Kontaktoberfläche zwischen dem wässrigen Medium 116 und dem Substratgas und desto größer die Reaktionsrate der Umwandlung. Das Einleiten des Substratgases über die Eingangsleitung 102 kann auch, abweichend von der Darstellung in Fig.4, unterhalb der Oberfläche des wässrigen Mediums 116 im Sprühturm 402 erfolgen, beispielsweise über eine Austrittsöffnung oder eine entsprechende Anordnung aus dicht beieinander angeordneten Austrittsöffnungen, so dass das Substratgas zunächst durch das im Sprühturm 402 als Flüssigkeitssäule vorliegende wässrige Medium 116 hindurchtreten kann und auch eine Methangasproduktion bereits in dieser Flüssigkeitssäule stattfinden kann. Generell können die in dieser Anmeldung vorgestellten Reaktionssteigerungsvorrichtungen in beliebiger zweckmäßiger Weise miteinander kombiniert werden, um die Kontaktoberfläche zwischen dem Substratgas und dem wässrigen Medium 116 und damit den methanogenen Mikroorganismen zu maximieren, um die Reaktionsrate der Methanproduktion zu maximieren.

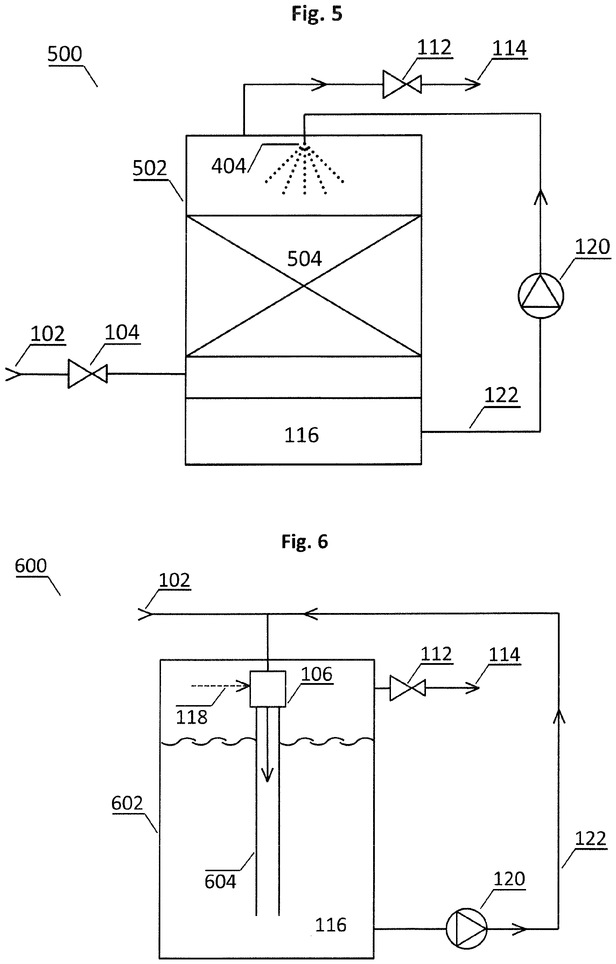

In **Fig.5** ist eine weitere Ausführungsform der Vorrichtung zum Herstellen von Methan mittels methanogener Mikroorganismen gezeigt, bei welcher ein Rieselbett-Reaktor 502 zum Einsatz kommt. Die Vorrichtung 500 ähnelt vom Aufbau her der in Fig.4 gezeigten Vorrichtung. Zusätzlich ist jedoch im Inneren eine poröse oder wabenartige Matrix 504 bereitgestellt, an der das von oben her eingesprühte flüssige Medium 116 herunterrieseln kann. Die Innenfläche der Matrix 504, beispielsweise die Größe der Poren oder Waben in ihrem Inneren, definiert dabei die Reaktionsoberfläche. Die Matrix 504 kann mit einem Material beschichtet oder aus diesem bestehen, beispielsweise Glas, Kunststoff, Lavaasche, welches eine gleichmäßige und flächendeckende Benetzung der Matrix 504 gewährleistet und/oder ein zu rasches Durchsickern des wässrigen Mediums 116 durch die Matrix 504 verhindert. Bei der in Fig.5 gezeigten Vorrichtung 500 zum Herstellen von Methan wird die Reaktionssteigerungsvorrichtung von der Matrix 504 und der Dispersionsvorrichtung 404 gebildet, da das Zusammenspiel dieser beiden Vorrichtungen im Vergleich zum statischen Fall eine vielfach vergrößerte Reaktionsoberfläche zwischen dem wässrigen Medium 116 und dem Substratgas ermöglicht.

Eine noch weitere Ausführungsform der Vorrichtung zum Herstellen von Methan mittels methanogener Mikroorganismen ist in **Fig.6** dargestellt, bei welcher ein Triebstrahlreaktor 602 verwendet wird. Hierbei wird von Außen das Substratgas und das wässrige Medium 116 der an der Decke bzw. im oberen Bereich des Triebstrahlreaktors 602 angeordneten Düse 106, beispielsweise einer Triebstrahldüse, zugeführt. Die Düse 106 kann ferner direkt die gasförmige Phase aus dem Inneren des Triebstrahlreaktors 602 ansaugen, was dem optionalen, ersten Rückführungszweig 118 entspricht. Der von der Düse 106 erzeugte Triebstrahl kann in das wässrige Medium 116 eingebracht bzw. eingestrahlt werden, wodurch es zu dessen turbulenter Durchmischung mit dem Substratgas kommt. Beispielsweise kann der Triebstrahl mittels eines Rohres 604 in das wässrige Medium 116 eingebracht werden, wobei an den Seiten des Rohres 604 feine Öffnungen vorgesehen sein können, durch die bereits ein Teil des auf dem Weg nach unten strömenden Substratgases in das wässrige Medium 116 eingeleitet werden kann. Es kann vorteilhaft sein, wenn das Ende des länglichen Rohres 604 nahe am Boden des Triebstrahlreaktors 602 angeordnet ist, so dass der Triebstrahl durch einen verhältnismäßig großen Anteil des wässrigen Mediums 116 durchtritt.

In **Fig. 7** ist eine beispielhafte Ausführungsform einer Vorrichtung zum Herstellen von Methan (Formelzeichen CH₄) mittels methanogener Mikroorganismen durch Umsetzung von Wasserstoff (Formelzeichen H₂) und Kohlenstoffdioxid (Formelzeichen CO₂) dargestellt. Die Vorrichtung ist ein Submers-Reaktor. Erfindungsgemäß werden methanogene Makrokolonien, sogenannte Pellets in hohen Dichten in einen überwiegend mit Nährflüssigkeit gefüllten Submersbioreaktor gegeben. Die Versorgung der Organismen mit ihrem Substrat, einem Wasserstoff-Kohlendioxid-Gemisch, erfolgt über eine intensive Begasung der Nährflüssigkeit in dem sich die Organismen befinden. Dies wird erreicht indem der Reaktor unter mehr als atmosphärischen Druck (12) betrieben wird, um die Löslichkeit des Wasserstoffes zu erhöhen. Des Weiteren wird der Reaktor über eine interne Gasrezirkulation (4) durchmischt. Dabei verfügt der Reaktor über einen begasten Upstreamteil (A) und unbegasten Downstreamteil (B) verfügt. Das Gas aus der über der Flüssigkeitsphase liegenden Gasphase wird unter Druck in den begasten Teil eingebracht und damit eine Aufwärtsströmung eines Gas-Flüssigkeits-Pellet-Gemisches erzeugt. Im oberen Reaktorteil wird das Gas in die Gasphase abgegeben, so dass ein Pellet-Nährmedium-Gemisch im unbegasten Teil (B) nach unten zurück strömen kann. Die rezirkulierte Gasmenge (4) ist dabei pro Zeiteinheit vielfach größer als die Menge des dem Reaktor zugeführten Gasgemisches (3).

Mehrere solcher Submersreaktoren (R₁ bis Rₙ) können in Serie als Kaskade hintereinander betrieben werden (siehe **Fig. 8****,** **9 und 10**)**.** Dabei strömt eine bestimmte Gasmenge von den vorderen zu den jeweils dahinter liegenden Reaktoren. Werden die Reaktoren mit unterschiedlichen Gasdrücken betrieben, stellen sich unterschiedliche Bedingungen in den Reaktoren und eine Pfropfenströmung ein. Das gasförmige Substrat kann auf diese Weise weitgehend zu Methan umgesetzt werden. Dieses Verfahren kann einen Beitrag leisten, nicht nutzbare Spitzenproduktion elektrischer Energie zu nutzen, um diese in eine speicherbare Form zu überführen, in das energiereiche Methan. Dabei verbraucht das Verfahren Kohlendioxid, das nicht in reiner Form dargeboten werden muss. Im Vergleich mit chemischen Verfahren findet die erfindungsgemäße Methode der Biomethanisierung unter natürliche Bedingungen statt. Methanbakterien können über längere Zeiträume ohne Nährstoff auskommen. Sie kann damit flexible auf den Bedarf reagieren.
Bei allen hier vorgestellten Ausführungsformen der Vorrichtung zum Herstellen von Methan mittels methanogener Mikroorganismen kann das CO₂ aus einem Rauchgas, beispielsweise aus einer Müllverbrennungsanlage oder einem Kraftwerk, etwa einem Kohlekraftwerk, gewonnen werden. Dazu kann die erfindungsgemäße Vorrichtung eine Anreicherungsvorrichtung aufweisen, welche beispielsweise einen mit Wasser gefüllten Druckbehälter aufweist, durch welchen Rauchgas durchgeleitet wird. Da CO₂ eine im Vergleich zu N₂ und O₂ bessere Löslichkeit aufweist, kann so das Wasser mit CO₂ unter Druck angereichert werden. Durch eine Drucksenkung kann dann zunähst das Rauchgas aus der Anreicherungsvorrichtung entfernt werden. Durch eine weitere Drucksenkung kann das CO₂ schließlich aus dem Wasser gelöst werden und als Substratgas der Düse 106 zugeführt werden und beispielsweise zusammen mit dem rezirkulierten wässrigen Medium 116 zur Bildung des Triebstrahls verwendet werden. Reste von Sauerstoff können vor Zuführung zur Düse 106 chemisch oder biologisch entfernt werden.

Im Vergleich mit chemischen Verfahren durch Katalyse, kann das erfindungsgemäße Verfahren auch mit nicht reinen Gasen durchführt werden. Schritte zur Reinigung der Gase entfallen. Im Vergleich zu anderen Methoden zu Biomethanisierung können hier gezielt definierte Biomassedichten eingestellt und bei Bedarf wieder hergestellt werden. Des Weiteren wird durch die interne Rezirkulation des Gases eine gute Gasversorgung der Organismen gewährleistet.

### Ausführunsqbeispiel 1

Wasser im Reaktor mit Nährsalze für Süßwassermedium nach Widdel (1980) sowie einem Promille Spurenelemente nach Immhoff-Stuckle et al. (1983) beschicken. Wasser im Reaktor auf ca. 60 - 70 °C erhitzen, Atmosphäre mit N₂ verdrängen. Reaktor verschließen. Wasser unter N₂ Atmosphäre auf ca. 40°C abkühlen lassen und dabei N₂ bis zu einem Druck von ca. 0,2 bar nachliefern. Ein Promille Vitaminlösung nach Balch et.al. (1979) zugeben. Mit NaS bis auf < -0,2 V reduzieren. Aktive, methanogene Bakterienmakrokolonien, sogenannte Pellets, aus einer anaeroben Industrieabwasserreinigungsanlage (z.B. Papierfabrik oder Lebensmittelindustrie) in ausreichender Konzentration zugeben (ca. 1% Trockensubstanz). Temperaturen von 35 bis 38°C und pH auf ca. 7 einstellen. Durchmischung des Rektors starten. Den Reaktor pro Zeiteinheit mit einer definierten Menge Substratgase (H₂/CO₂) in einem Verhältnis ca. 4:1 mit einem Druck von 0,2 bar beschicken. Im Reaktor einen Druck von 0,2 - 0,1 bar halten. Das entstehendes Gasgemisch aus dem Reaktor bei einem Druck von > 0,2 - 0,1 bar ablassen.

### Literatur

- Balch, W.E.; Fox, G.E. Magrum, L.J.; Woese, C.R. ; Wolfe, R.S. (1979): Methanogenis: Reevaluation of a unique biological group. Micobiol. Review 43, 260 -296
- Bajohr et al.: Speicherung von regenerativ erzeugter elektrischer Energie in der Ergasinfrastruk-tur, DVGW- Studie, 2011
- Brunner, M. (1987): "Anaerober mikrobieller Abbau von 3-Chlor-Benzoesäure zu Biogas" (1987), Inaugural-Dissertation zur Erlangung des Doktorgrades der Mathematisch-Naturwissenschaftlichen Fakultät der Universität Düsseldorf
- Brunner, M.; Schoberth, S.M.; Sahm, H. (1987): Anaerober mikrobieller Abbau von halogenierten Aromaten zu Biogas. Chem.-Ing.-Tech. 59 (1), 55-57
- Brunner, M. (1989): "Neue Erfahrungen mit der anaeroben Abwasserreinigung mittels UASB-Reaktoren in der Papierindustrie", Allgemeine Papierrundschau 11/1989
- Brunner, M. , Dietrich, P. (1988):" Konzepte zur anaeroben Abwasserreinigung in der Fruchtsaftindustrie", Confructra 32 III/IV, 118 - 125
- Das Erdgasnetz als Systemintegrator zur Verstetigung von Wind- und Solarstrom, energie - wasser-praxis, 2010
- DVGW-Arbeitsblatt G 262 "Nutzung von Gasen aus regenerativen Quellen in der öffentlichen Gasversorgung", November 2004
- Energiespeicher in Stromversorgungssystemen mit hohem Anteil erneuerbarer Energieträger, Energietechnische Gesellschaft im VDE, Frankfurt, 2009
- Erneuerbare Energien in Zahlen - Nationale und Internationale Entwicklung, Bundesministeriums für Umwelt, Naturschutz und Reaktorsicherheit, 2010
- George A. Olah et al (2008): Chemical Recycling of Carbon Dioxide to Methanol and Dimethyl Ether Loker Hydrocarbon Research Institute and Department of Chemistry, University of Southern Cali-fornia, 2008
- Guiot et al. (2011): Potential of wastewater-treating anaerobic granules for biomethanatioon of synthesis gas", Environmental Science & Technology Vol. 45, 2006-2012
- Hartmut Wendt (1984): Neue konstruktive und prozesstechnische Konzepte für die Wasserstoffgewinnung durch Elektrolyse, Chem. Ing. Tech 56, 1984
- Immhof-Struckle, D. und Pfennig, N. (1983): Isolation and characterisation of a nicotinic acid-degrading sulfat-reducing bacterium, desulfococcus niacini. Arch. Microbiol. 136, 194-198
- Kopyscinski et al: Production of synthetic natural gas (SNG) from coal and dry biomass - A technology review from 1950 to 2009, General Energy Research Department, Paul Scherrer Institut, Villingen, Schweiz
- Klaus, T. et al. (2010): Energieziel 2050: 100% Strom aus erneuerbaren Quellen, Umwelt Bundesamt, Dessau-Roßlau 2010
- Marcus Reppich: Vergleich verschiedener Aufbereitungsverfahren von Biogas zur Einspeisung in das Erdgasnetz, Chem. Ing. Tech. 81 Nr.3, 2009
- Pehnt, Martin; Höpfner Ulrich: Kurzgutachten Wasserstoff- und Stromspeicher in einem Energie-system mit hohen Anteilen erneuerbarer Energien: Analyse der kurz- und mittelfristigen Perspek-tive, ifeu -Institut für Energie und Umweltforschung Heidelberg GmbH, Im Auftrag des Bundesmi-nisteriums für Umwelt, Naturschutz und Reaktorsicherheit (BMU), Heidelberg, Mai 2009; V. 1.1
- Power to Gas: Untersuchungen im Rahmen der DVGW-Innovationsoffensive zur Energiespeiche-rung, energie I wasser-praxis, 2011
- Sahm, H. (1981): Biologie der Methanbildung, Chem.-Ing. Tech. 53 (11), 854-863
- Sahm, H. (1984): Anaerobic wastewater treatment. Ad. Biochem. Eng. Biotech. 29, 83-115
- Sahm, H.; Brunner, M.; Schoberth, S.M. (1986): Anaerobic degradation of halogenated aromatic compounds. Microbial Ecology 12, 147-153,
- Schoberth, S.M., Brunner, M., Sahm, H. (1984):"Anaerobic degradation of 3-chlorbenzoic acid to methan in a defind medium", IAWPRC Symposium on Forest Industry Waste Water, Tamper
- Schoberth, S.M., Brunner, M., Sahm, H. (1988):"Anaerober Abbau von Halogenaromaten" , Gas-Wasser-Fach 129, (1), 32 - 34
- Sterner, M.(2009): Dynamische Simulation der Stromversorgung in Deutschland nach dem Ausbauszenario der Erneuerbaren-Energien-Branche, Abschlussbericht, Fraunhofer IWES, 2009
- Sterner, M. (2009): Bioenergy and renewable power methane in integrated 100% renewable energy systems, Limiting global warming by transforming energy systems, Dissertation, Universität Kassel, Fraunhofer IWES, 2009
- Sterner, M.; Specht, M. (2010): Erneuerbares Methan. Eine Lösung zur Integration und Speicherung erneuerbarer Energien und ein Weg zur regenerativen Vollversorgung, In Solarzeitalter 1/2010
- Sterner, M. (2011): Energiewirtschaftliche und ökologische Bewertung des Windgas-Angebotes, Fraunhofer IWES, Februar 2011
- Tom Smolinka et al. (2011): NOW Studie, Stand und Entwicklungspotential der Wasserelektrolyse zur Herstellung von Wasserstoff aus regenerativen Energien, Kurzfassung des Abschlussberichts, 2011
- Vereijken, T., Brunner, M. (1989): "Neue Entwicklungen in der Brauereiabwasserbehandlung", Brauindustrie 6 /1989, 653 - 656
- Verband der Schweizerischen Gasindustrie (VSG),A EE Agentur für Erneuerbare Energien und Energieeffizienz: "Swiss Renewable Power-to-Gas - Erneuerbares Gas aus Strom für die Schweiz", Bern, Juni 2012
- Widdel, F. (1980): Anaerober Abbau von Fettsäuren und Benzoesäure durch neu isolierte Sulfat-reduzierende Bakterien. Dissertation, Universität Göttingen

## Patentansprüche

1. Vorrichtung zum Herstellen von Methan mittels methanogener Mikroorganismen durch Umsetzung von H₂ und CO₂, aufweisend:
- mindestens einen Reaktor;
- ein wässriges Medium, welches in dem mindestens einen Reaktor bereitgestellt ist, wobei sich die methanogenen Mikroorganismen in dem wässrigen Medium befinden;
- eine Zuführungseinrichtung, welche zum Einleiten von H₂ und CO₂ in den mindestens einen Reaktor eingerichtet ist, wobei das H₂ und CO₂ darin ein gasförmiges Gemisch bilden;
- eine Reaktionssteigerungsvorrichtung, welche eingerichtet ist zum Vergrößern der Kontaktoberfläche zwischen dem wässrigen Medium mit den methanogenen Mikroorganismen und dem gasförmigen Gemisch;
- eine Rückführungseinrichtung, welche eingerichtet ist zum Rückführen mindestens eines Teils des sich im Reaktor ansammelnden Gases, wobei die Rückführungsrate des sich im Reaktor ansammelnden Gases größer ist als die Zuführungsrate des Substratgases in den Reaktor.

2. Vorrichtung nach Anspruch 1, ferner aufweisend:
eine Abführeinrichtung, welche zum Abführen eines Gases aus dem mindestens einen Reaktor eingerichtet ist.

3. Vorrichtung nach Anspruch 1 oder 2, wobei das Gas Methan aufweist und wobei vorzugsweise der Stoffmengenanteil von Methan in dem Gas mindestens 50% beträgt.

4. Vorrichtung nach einem der Ansprüche 1 bis 3
wobei die Rückführungsrate des sich im Reaktor ansammelnden Gases die Zuführungsrate des Substratgases in den Reaktor um mindestens den Faktor 100 übersteigt.

5. Vorrichtung nach einem der Ansprüche 1 bis 4, wobei die Rückführungseinrichtung eine Pumpe oder einen Kompressor aufweist.

6. Vorrichtung nach einem der Ansprüche 1 bis 5, ferner aufweisend:
eine Zirkulationseinrichtung, die eingerichtet ist zum Zirkulieren des wässrigen Mediums durch den mindestens einen Reaktor, wobei die Zirkulationseinrichtung optional mindestens eine Pumpe, vorzugsweise Triebstrahlpumpe aufweist.

7. Vorrichtung nach einem der Ansprüche 1 bis 6, wobei die Vorrichtung als stationärer oder instationärer Reaktor, vorzugsweise Feststoffreaktor oder optional als ein Submers-Reaktor eingerichtet ist.

8. Vorrichtung nach einem der Ansprüche 1 bis 7, wobei die Vorrichtung als kontinuierlicher, idealer Rührkessel (CSTR), diskontinuierlicher, idealer Rührkessel (STR), Rohrreaktor, als Schlaufenreaktor, als in Serie geschaltete Reaktoren oder als Kaskade von Reaktoren eingerichtet ist; und
wobei die Reaktionssteigerungsvorrichtung bevorzugt mindestens ein Rieselbett, einen Sprühturm, einen Rohrmischer oder eine Pumpe, vorzugsweise eine Triebstrahlpumpe aufweist.

9. Vorrichtung nach einem der Ansprüche 1 bis 8, wobei die Zuführungseinrichtung ferner aufweist:
eine Vorrichtung zum Anreichern von Kohlendioxid aus einem Gasgemisch; und
optional eine Einrichtung zum elektrolytischen Spalten von Wasser;
wobei die Zuführeinrichtung bevorzugt derart eingerichtet ist, das beim Einleiten des H₂ und CO₂ in den mindestens einen Reaktor das gasförmige Gemisch eine turbulente Strömung bildet.

10. Vorrichtung nach einem der Ansprüche 1 bis 9, wobei die Zuführungseinrichtung ferner derart eingerichtet ist, dass im Betrieb das Volumen des eingeleiteten H₂ und CO₂ pro Stunde das Fassungsvolumen des mindestens einen Reaktors um mindestens den Faktor 2 übersteigt; wobei der Druck im Reaktor optional größer oder gleich 0,1 bar ist;
wobei sich die methanogenen Mikroorganismen bevorzugt in Form von makroskopischen Kolonien (Pellets) in dem wässrigen Medium befinden; und
wobei ferner optional die methanogenen Mikroorganismen in dem wässrigen Medium nicht immobilisiert sind.

11. Verfahren zur Herstellung von Methan in einer Reaktorvorrichtung mittels methanogener Mikroorganismen, die sich in der Reaktorvorrichtung in einem wässrigen Medium befinden, durch Umsetzung von H₂ und CO₂ als Reaktionsgasgemisch, wobei bei der Umsetzung die Kontaktoberfläche zwischen dem wässrigen Medium mit den methanogenen Mikroorganismen und dem gasförmigen Gemisch mittels einer Reaktionssteigerungsvorrichtung vergrößert wird, **dadurch gekennzeichnet, dass** mittels einer Rückführungseinrichtung mindestens ein Teils des sich im Reaktor ansammelnden Gases in die Reaktorvorrichtung zurückgeführt wird, wobei die Rückführungsrate des sich im Reaktor ansammelnden Gases größer ist als die Zuführungsrate des Reaktionsgemisches in den Reaktor.

12. Verfahren nach Anspruch 11, **dadurch gekennzeichnet, dass** die methanogenen Mikroorganismen ein Gemisch verschiedener Mikroorganismen des Stamms Euryarchaeota sind und, dass die methanogenen Mikroorganismen bevorzugt in Form von makroskopischen Kolonien (Pellets) dem wässrigen Medium zugeführt werden.

13. Verfahren nach einem der Ansprüche 11 bis 12, **dadurch gekennzeichnet,** das s das molare Verhältnis von H₂ und CO₂ im gasförmigen Gemisch kleiner oder gleich 4 : 1 ist.

14. Verwendung einer Vorrichtung nach einem der Ansprüche 1 bis 10 zur Herstellung von Methan mittels methanogener Mikroorganismen durch Umsetzung von H₂ und CO₂.

15. Verwendung von Pellets, enthaltend methanogene Mikroorganismen, zum Beimpfen einer Vorrichtung nach einem der Ansprüche 1 bis 10.

## Claims

1. A device for the production of methane by means of methanogenic microorganisms through conversion of H₂ and CO₂, having:
- at least one reactor;
- an aqueous medium, which is prepared in the at least one reactor, the methanogenic microorganisms being located in the aqueous medium;
- a feed mechanism, which is designed to channel H₂ and CO₂ into the at least one reactor, wherein the H₂ and CO₂ form a gaseous mixture inside it;
- a reaction boosting device, which is designed to increase the contact surface between the aqueous medium with the methanogenic microorganisms and the gaseous mixture;
- a return mechanism, which is designed to return at least a portion of the gas, which accumulates in the reactor, wherein the return rate of the gas accumulating in the reactor is greater than the feed rate of the substrate gas in the reactor.

2. The device of claim 1, moreover having:
a drainage mechanism, which is designed to drain a gas from the at least one reactor.

3. The device of claim 1 or 2, wherein the gas has methane and preferably wherein the quantitative fraction of methane in the gas is at least 50%.

4. The device of any one of claims 1 to 3, wherein the return rate of the gas accumulating in the reactor exceeds the feed rate of the substrate gas in the reactor by at least the factor of 100.

5. The device of any one of claims 1 to 4, wherein the return mechanism has a pump or a compressor.

6. The device of any one of claims 1 to 5, moreover having:
a circulation mechanism, which is designed to circulate the aqueous medium through the at least one reactor, wherein the circulation mechanism optionally has at least on pump, preferably a driving jet pump.

7. The device of any one of claims 1 to 6, wherein the device is set up as stationary or nonstationary reactor, preferably a solid state reactor or optionally as a submerse reactor.

8. The device of any one of claims 1 to 7, wherein the device is set up as continuous ideal stirred tank (CSTR), discontinuous ideal stirred tank (STR), tube reactor, loop reactor, reactors switched in series, or as cascade of reactors; and
wherein the reaction boosting device preferably has at least one trickling bed, one spray tower, one in-line mixer or one pump, preferably a driving jet pump.

9. The device of any one of claims 1 to 8, wherein the feed mechanism moreover has:
a device for enrichment of carbon dioxide from a gas mixture; and
optionally a mechanism for electrolytic splitting of water;
wherein the feed mechanism preferably is designed so that when the H₂ and CO₂ are channeled into the at least one reactor, the gaseous mixture forms a turbulent flow.

10. The device of any one of claims 1 to 9, wherein the feed mechanism moreover is designed so that, in operation, the volume of introduced H₂ and CO₂ per hour exceeds the volume capacity of the at least one reactor by at least the factor of 2, wherein the pressure in the reactor is optionally greater than or equal to 0.1 bar;
wherein the methanogenic microorganisms preferably are present in form of macroscopic colonies (pellets) in the aqueous medium; and
wherein moreover optionally the methanogenic microorganisms are not immobilized in the aqueous medium.

11. A method for production of methane in a reactor device by means of methanogenic microorganisms, which are located in the reactor device in an aqueous medium, by conversion of H₂ and CO₂ as reaction gas mixture, wherein the contact surface between the aqueous medium with the methanogenic microorganisms and the gaseous mixture is increased by means of a reaction boosting device during the conversion, **characterized in that** by means of a return mechanism at least a portion of the gas, which accumulates in the reactor, is returned in the reactor device, wherein the return rate of the gas accumulating in the reactor is greater than the feed rate of the reaction mixture in the reactor.

12. The method of claim 11, **characterized in that** the methanogenic microorganisms are a mixture of different microorganisms of the phylum Euryarchaeota and, that the methanogenic microorganisms preferably in form of macroscopic colonies (pellets) are supplied to the aqueous medium.

13. The method of any one of claims 11 to 12, **characterized in that** the molar ratio of H₂ and CO₂ in the gaseous mixture is less than or equal to 4 : 1.

14. Use of a device according to any one of claims 1 to 10 for the production of methane by means of methanogenic microorganisms by conversion of H₂ and CO₂.

15. Use of pellets containing methanogenic microorganisms for seeding of a device according to any one of claims 1 to 10.

## Revendications

1. Dispositif de production de méthane par des micro-organismes méthanogènes par réaction de H2 et CO2, comprenant:
au moins un réacteur;
un milieu aqueux prévu dans ledit au moins un réacteur, lesdits microorganismes méthanogènes étant présents dans ledit milieu aqueux;
un dispositif d'alimentation agencé pour introduire H₂ et CO₂ dans ledit au moins un réacteur, lesdits H₂ et CO₂ y formant un mélange gazeux;
un dispositif d'augmentation de réaction conçu pour augmenter la surface de contact entre le milieu aqueux avec les microorganismes méthanogènes et le mélange gazeux;
un dispositif de recyclage agencé pour recycler au moins une partie du gaz qui s'accumule dans le réacteur, dans laquelle le taux de recyclage du gaz s'accumulant dans le réacteur est supérieur au taux d'alimentation du gaz substrat dans le réacteur.

2. Dispositif selon la revendication 1, comprenant en outre:
un dispositif de décharge agencé pour décharger un gaz à partir dudit au moins un réacteur.

3. Dispositif selon les revendications 1 ou 2, dans lequel le gaz contient du méthane et dans lequel la quantité de méthane dans le gaz est de préférence d'au moins 50%.

4. Dispositif selon l'une des revendications 1 à 3,
dans laquelle le taux de recirculation du gaz s'accumulant dans le réacteur dépasse le taux d'alimentation du gaz substrat dans le réacteur d'au moins un facteur 100.

5. Dispositif selon l'une quelconque des revendications 1 à 4, le dispositif de recyclage comprenant une pompe ou un compresseur.

6. Dispositif selon l'une des revendications 1 à 5, comprenant en outre:
un dispositif de circulation qui est agencé pour faire circuler le milieu aqueux à travers ledit au moins un réacteur, dans lequel le dispositif de circulation comprend éventuellement au moins une pompe, de préférence une pompe à jet d'entraînement.

7. Dispositif selon l'une quelconque des revendications 1 à 6, le dispositif étant agencé sous forme d'un réacteur fixe ou transitoire, de préférence un réacteur solide, ou éventuellement un réacteur submersible.

8. Dispositif selon l'une quelconque des revendications 1 à 7, le dispositif étant agencé sous la forme d'une cuve d'agitation idéale continue (CSTR), d'une cuve d'agitation idéale discontinue (STR), d'un réacteur tubulaire, d'un réacteur en boucle, d'un réacteur en série ou en cascade ; et
le dispositif d'augmentation de la réaction comportant de préférence au moins un lit à ruissellement, une tour de pulvérisation, un mélangeur tubulaire ou une pompe, de préférence une pompe à jet d'entraînement.

9. Dispositif selon l'une quelconque des revendications 1 à 8, ledit dispositif d'alimentation comprenant en outre: dispositif d'enrichissement en dioxyde de carbone d'un mélange gazeux ; et éventuellement dispositif de craquage électrolytique de l'eau; dans laquelle le dispositif d'alimentation est disposé de préférence de telle sorte que, lorsque le H₂ et le CO₂ sont introduits dans ledit au moins un réacteur, le mélange gazeux forme un écoulement turbulent.

10. Dispositif selon l'une quelconque des revendications 1 à 9, lesdits moyens d'alimentation étant en outre agencés de telle sorte que, lors de l'utilisation, le volume de H₂ et de CO₂ injecté par heure dépasse le volume de rétention dudit au moins un réacteur d'au moins un facteur 2 ; dans lequel la pression dans le réacteur est éventuellement supérieure ou égale à 0,1 bar;
dans lequel les microorganismes méthanogènes sont de préférence sous forme de colonies macroscopiques (pastilles) dans le milieu aqueux; et dans lequel en outre les microorganismes méthanogènes ne sont éventuellement pas immobilisés dans le milieu aqueux.

11. Procédé de production de méthane dans un dispositif de réacteur par des microorganismes méthanogènes en milieu aqueux dans le dispositif de réacteur par réaction de H₂ et CO₂ sous forme de mélange gazeux de réaction, Dans lequel lors de la réaction, la surface de contact entre le milieu aqueux avec les microorganismes méthanogènes et le mélange gazeux est agrandie à l'aide d'un dispositif d'augmentation de la réaction, **caractérisé en ce qu'**au moins une partie du gaz accumulé dans le réacteur est renvoyée dans le dispositif du réacteur au moyen d'un dispositif de recyclage, dans lequel le taux de recyclage du gaz s'accumulant dans le réacteur est supérieur au taux d'alimentation du gaz substrat dans le réacteur.

12. Procédé selon la revendication 11, **caractérisé en ce que** les microorganismes méthanogènes sont un mélange de différents microorganismes de la souche Euryarchaeota et **en ce que** les microorganismes méthanogènes sont de préférence amenés au milieu aqueux sous forme de colonies macroscopiques (pastilles).

13. Procédé selon l'une des revendications 11 à 12, **caractérisé en ce que** le rapport molaire de H₂ et CO₂ dans le mélange gazeux est inférieur ou égal à 4:1.

14. Utilisation d'un dispositif selon l'une quelconque des revendications 1 à 10 pour la production de méthane par des micro-organismes méthanogènes par réaction de H₂ et CO₂.

15. Utilisation de pastilles contenant des micro-organismes méthanogènes pour inoculer un dispositif selon l'une des revendications 1 à 10.
